(19) Europäisches Patentamt
European Patent Office
Office européen des brevets

(11) Numéro de publication: **0 346 208 B1**

# (12) FASCICULE DE BREVET EUROPEEN

(45) Date de publication de fascicule du brevet: **13.04.94**

(51) Int. Cl.[5]: **C07D 215/54**, C07D 215/42, C07D 215/60, C07D 471/04, C07D 401/06, A61K 31/47, A61K 31/44, //(C07D471/04, 221:00,221:00)

(21) Numéro de dépôt: **89401548.6**

(22) Date de dépôt: **05.06.89**

## (54) Amino-4 quinoléines et naphtyridines, leur procédé de préparation et leur application comme médicaments.

(30) Priorité: **06.06.88 FR 8807498**
**15.06.88 FR 8808025**

(43) Date de publication de la demande:
**13.12.89 Bulletin 89/50**

(45) Mention de la délivrance du brevet:
**13.04.94 Bulletin 94/15**

(84) Etats contractants désignés:
**AT BE CH DE ES FR GB GR IT LI LU NL SE**

(56) Documents cités:
**EP-A- 0 018 735**
**EP-A- 0 205 362**
**EP-A- 0 245 054**

**CHEMICAL ABSTRACTS, vol. 97, no. 19, 8 novembre 1982, page 706, résumé no. 162862n, Columbus, Ohio, US; R.M. TITKOVA et al.: "Synthesis of 4-substituted 3-carbethoxy[carboxy]-1,5-naphthyridines, their properties and biological activity"**

(73) Titulaire: **ELF SANOFI**
**32-34, rue Marbeuf**
**F-75008 Paris(FR)**

(72) Inventeur: **Mendes, Etienne**
**2, place Jeanne d'Arc**
**F-31000 Toulouse(FR)**
Inventeur: **Vernieres, Jean-Claude**
**rue Sabatié Garat**
**F-31600 Muret(FR)**
Inventeur: **Keane, Peter Eugène**
**8, rue Marcel Doret, Roquettes**
**F-31120 Portet-sur-Garonne(FR)**
Inventeur: **Bachy, André**
**2, Impasse Professeur Astre**
**F-31100 Toulouse(FR)**

(74) Mandataire: **Polus, Camille et al**
**c/o Cabinet Lavoix**
**2, Place d'Estienne d'Orves**
**F-75441 Paris Cedex 09 (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 346 208 B1

Rank Xerox (UK) Business Services
(3.10/3.09/3.3.3)

## Description

La présente invention concerne de nouveaux dérivés d'amino-4 quinoléines et naphtyridines, leur procédé de préparation et leur application en thérapeutique.

On connaît déjà des dérivés de naphtyridines et surtout de quinoléines utiles en thérapeutique pour des activités pharmacologiques très diverses, telles que antibactériennes, antihypertensives, anxiolytiques, anti-inflammatoires, et analgésiques, activités dépendant essentiellement des groupes substituant les noyaux aromatiques. Parmi ceux-ci, quelques dérivés ont été signalés comme se fixant sur les récepteurs de benzodiazépines, on peut citer notamment :

- les amino-4 carbamoyl-3 quinoléines, décrites dans EP-A-0 245 054, de formule A :

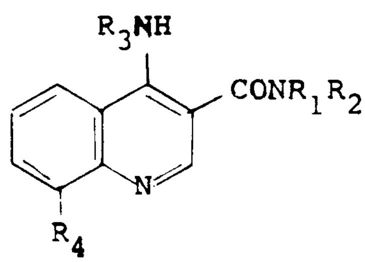

A

dans laquelle $R_1$, $R_2$, $R_3$, $R_4$ sont H ou des groupes hydrocarbonés : alkyle, aryle, aralkyle;

- les quinoléines, décrites dans FR-A-2 582 514, de formule B

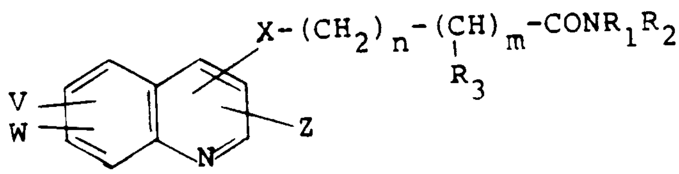

B

dans laquelle $R_1$, $R_2$, $R_3$, $R_4$ représentent H ou des groupes alkyles ou aryles, Z représente un groupe aryle et X représente $CH-R_4$, $N-R_4$, SO, $SO_2$, O ou S et V et W représentent H, halogène, alkyle, alkoxy, $NO_2$ ou $CF_3$;

- les naphtyridines, décrites dans EP-A-0 234 971, de formule C

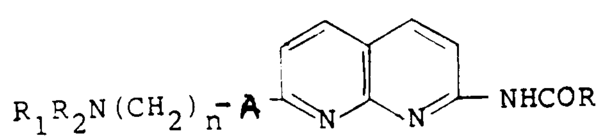

C

dans laquelle R représente un groupe cycloalkyle, hétéroaryle, phényle substitué ou non, A représente N, S, SO, ou O et $R_1$, $R_2$ représentent notamment H ou un groupe alkyle:

- les quinoléines, décrites dans FR-A-2 581 382, de formule D

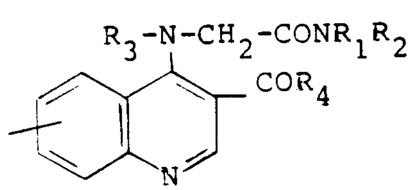

D

dans laquelle $R_1$, $R_2$, $R_3$ représentent H, un groupe alkyle ou aryle, et $R_4$ représente OH, un groupe alkoxy ou alkyle.

On a maintenant trouvé de nouveaux composés qui ne se fixent pas, à des doses pharmacologiquement significatives, sur les récepteurs centraux des benzodiazépines comme ces composés, mais se fixent uniquement sur Les récepteurs de type périphérique dont on sait que l'occupation entraîne des activités pharmacologiques différentes de celles résultant de l'activation des récepteurs centraux.

L'invention concerne les composés répondant à la formule I

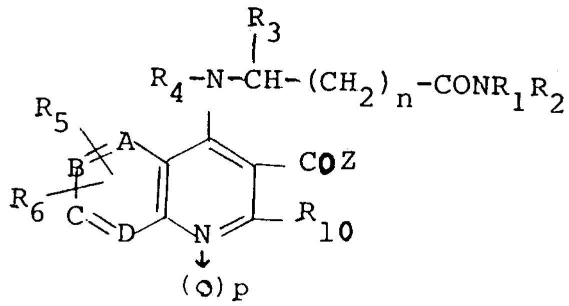

I

dans laquelle

$R_1$ et $R_2$, identiques ou différents, représentent chacun l'atome d'hydrogène, un groupe alkyle en $C_1$ à $C_6$ ou alkényle en $C_2$ à $C_6$, un groupe phényle ou benzyle, ou $R_1$ et $R_2$ forment avec l'atome d'azote auquel ils sont attachés un hétérocyle saturé choisi parmi pipéridine, pyrrolidine, morpholine et pipérazine,

$R_3$ représente l'atome d'hydrogène, un groupe alkyle en $C_1$ à $C_6$, phénylalkyle en $C_7$ à $C_9$, ou phényle,

$R_4$ représente l'atome d hydrogène ou un groupe alkyle en $C_1$ à $C_4$,

$R_5$ et $R_6$, identiques ou différents représentent chacun un atome d'hydrogène ou d'halogène, un groupe alkyle ou alkoxy en $C_1$ à $C_3$, un groupe nitro trifluorométhyle, ou ensemble forment un groupe méthylènedioxy,

Z représente $OR_7$ et $R_7$ représente l'atome d'hydrogène ou un groupe alkyle en $C_1$ à $C_6$ : $NR_8R_9$ et $R_8$ et $R_9$ identiques ou différents représentent chacun l'atome d hydrogène un groupe alkyle en $C_1$ à $C_4$ un groupe phényle ou benzyle; un groupe alkyle en $C_1$ à $C_4$ : un groupe benzyle ou un groupe aryle choisi parmi les groupes phényle, pyridyle, pyrrolyle, furyle, thiényle, et imidazolyle.

$R_{10}$ représente l'atome d'hydrogène, un groupe alkyle en $C_1$ à $C_4$ ou un groupe phényle ;

n représente 0,1 ou 2; p représente 0 ou 1; et

l'un des symboles A,B,C,D représente N et les autres CH ou A,B,C,D représentent chacun CH,

étant entendu que lorsque Z n'est pas un groupe aryle ou benzyle, $R_3$ ne représente pas H

ainsi que leurs sels d'addition avec les acides ou les bases pharmaceutiquement acceptables.

Parmi les acides, on peut citer les acides halogénhydriques, nitrique, sulfurique, phosphorique ou les acides carboxyliques tels que l'acide acétique, formique, succinique, tartrique, oxalique et aspartique ou les acides sulfoniques tels que l'acide méthanesulfonique ou benzénesulfonique; les sels avec les bases peuvent être des sels alcalins, alcalino-terreux ou des sels avec des amines telles que la lysine, la piperazine ou l'éthanolamine.

Les groupes alkyles peuvent être linéaires, ramifiés ou cycliques.

Les groupes phényles ou benzyles peuvent être substitués par les halogènes, les groupes alkoxy, alkyles ou thioalkyles en $C_1$ à $C_3$, les groupes nitro, trifluorométhyle ou hydroxyle.

Lorsqu'il existe un carbone assymétrique, les racémiques et les stéréoisomères sont objets de l'invention.

Parmi les produits préférés objets de l'invention, on peut citer les esters de quinoléine ou de naphtyridine-1,5 de formule I dans laquelle $R_{10}$ est H, $R_7$ représente un groupe alkyle en $C_1$ à $C_3$ et plus particulièrement éthyle, $R_3$ représente un alkyle en $C_1$ à $C_3$ et plus particulièrement le groupe méthyle, n est 0 et $R_4$ représente H et mieux ceux où le symbole C est substitué par un atome différent de H, ainsi que les composés pour lesquels Z représente un groupe aromatique ou hétéroaromatique, n est O et $R_4$ représente H et plus particulièrement parmi ceux-ci ceux dans lesquels $R_1$ représente un radical alkyle en $C_1$ à $C_4$ et $R_2$ représente un noyau phényle substitué ou non.

Deux autres groupes de composés préférés sont ceux de formule I dans laquelle :

- $R_1$ et $R_2$ représentent chacun un groupe alkyle en $C_1$ à $C_6$, $R_3$ représente un groupe alkyle en $C_1$ à $C_3$, $R_{10}$ représente l'hydrogène et Z représente $OR_7$ avec $R_7$ représentant un groupe alkyle en $C_1$ à $C_6$, et en particulier ceux pour lesquels $R_4$ est un hydrogène, ou ceux pour lesquels $R_4$ étant un hydrogène, le symbole C est substitué ; ou bien

- $R_1$ représente un groupe alkyle, $R_2$ et Z représentent chacun un groupe phényle pouvant être substitué, et en particulier ceux pour lesquels $R_3$ et $R_{10}$ représentent chacun l'atome d'hydrogène. Parmi les composés pour lesquels $R_1$ est un alkyle et $R_2$ et Z représentent chacun un groupe phényle pouvant être substitué, on préfère ceux pour lesquels les symboles A, B, C et D représentent chacun CH, et ceux pour lesquels le symbole A représente N et B, C et D représentent CH.

Un autre objet de l'invention est le procédé de préparation des composés de formule I qui consiste à faire réagir sur l'amine de formule II :

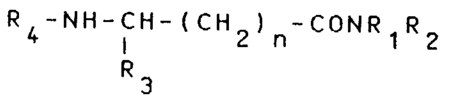

II

le dérivé de formule III

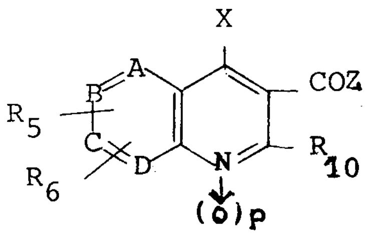

III

dans lesquelles $R_1$, $R_2$, $R_3$, $R_4$, $R_5$, $R_6$, $R_{10}$, Z, A, B, C, D, n et p ont les mêmes significations que dans la formule I et X représente un halogène tel que Cl ou Br ou un groupe sulfonate $RSO_3$ dans lequel R est un groupe alkyle ou benzyle.

La substitution de l'amine II peut être effectuée dans des conditions classiques, à une température comprise entre 80 °C et 180 °C et de préférence pour fixer l'acide formé en présence d une base organique ou minérale telle qu'une amine tertiaire ou un carbonate alcalin : on opère en général dans un solvant organique, tel qu'un hydrocarbure comme le toluène, tel qu'un alcool comme l'éthanol ou l'isopropanol, tel qu'un éther comme le dioxanne, ou tel qu'un solvant aprotique polaire, comme le diméthylformamide ; la réaction peut aussi être effectuée à une pression supérieure à la pression atmosphérique, comprise entre 5 et 70 x $10^5$ Pa, dans une enceinte close; on peut aussi effectuer la réaction en milieu diphasique, en présence d'un catalyseur de transfert de phase.

Certains dérivés de formule II ont déjà été décrits, tel que l'amino-2 N-N-diéthylpropanamide dans J. Chem. Soc. p. 2972-2980 (1952). Les autres peuvent être préparés à partir de produits connus en appliquant des méthodes classiques et par example :

- à partir des dérivés d'aminoacides IV, dont la fonction amine est protégée sous forme de carbamate labile et la fonction acide est activée par un groupe succinimidoyle ou p-nitrophénylcarboxy comme décrit par G.W. Anderson dans J.A.C.S. 86 p. 1839-1842 (1964) :

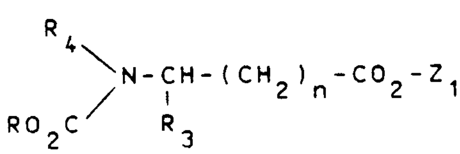

IV

dans laquelle $Z_1$ représente succinimidyl ou p-nitrophényl, $R_3$, $R_4$, et n ont les mêmes significations que dans la formule I et R représente un groupe alkyle en $C_1$ à $C_4$, sur lesquels on fait réagir l'amine HN $R_1$ $R_2$, pour donner le composé de formule V :

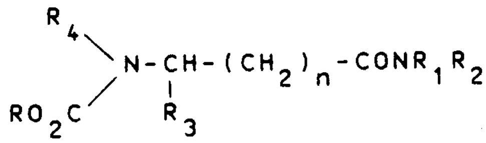

V

(dans laquelle $R_1$, $R_2$, $R_3$, $R_4$, et n ont la même signification que dans la formule I et R est un alkyle), avant d'éliminer le groupement carboxylique protégeant l'amine terminale par action d'un acide tel que les acides trifluoroacétique, sulfurique ou chlorhydrique;

- à partir d'amides de formule VI

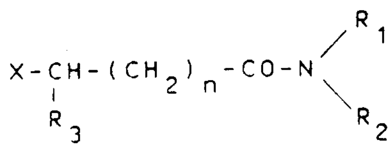

VI

(dans laquelle X représente Cl ou Br, et $R_1$, $R_2$, $R_3$ et n ont les mêmes significations qu'à la formule I) qui peuvent être préparés selon le procédé décrit dans Synthetic Organic Chemistry - chap. 19; R.B. Wagner et H.D. Zook (1953), publié par J. Wiley et Sons et pour n = O et $R_3$ = H par action de $HNR_1R_2$ sur $ClCH_2COCl$, sur lesquels on fait réagir l'amine $R_4$-N $H_2$, à une température comprise entre 60 °C et 130 °C, en solution dans un alcool ou un éther, à pression atmosphérique ou entre 5 et 40 x $10^5$ Pa, éventuellement en présence d'une base minérale ou organique; le mode opératoire peut être celui décrit par J.B.M. Bettolo et J.F. Cavalla dans Gazz. Chim. Ital. p. 896-907 (1954)

- par l'intermédiaire d'un phtalimide de formule VII lorsque $R_4$ = H,

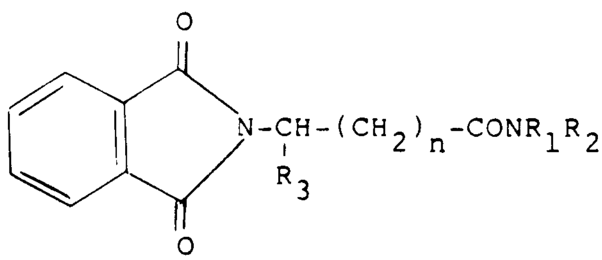

VII

selon une méthode analogue à celle décrite par R.D. Haworth et coll. dans J. Chem. Soc. p. 2972-2980 (1952).

Certaines quinoléines de départ ou les dérivés hydroxylés en 4 correspondants (formule III : A = B = C = D = CH; X = OH ou Cl) sont des composés connus décrits notamment dans J. Med. Chem <u>22</u>, 7 p. 816-823 (1979) ou dans FR-A-2 581 382, précédemment cité; et celles dans lesquelles $R_{10}$ est différent de H, peuvent être préparées par la méthode décrite par R.P. Staiger et E.B. Miller dans J. Org. Chem. <u>24</u>, p. 1214 (1959).

Les naphtyridines de formule III, dans lesquelles X = OH et Z représente $OR_7$ peuvent être préparées par des méthodes classiques, précédemment décrites, par example, dans Heterocyclic compounds - vol. 7 (1961) : naphtyridines (Chap. 2) Ed. J. Wiley.

Les nouveaux composés de formule III dans lesquels X = OH et Z représente un groupe alkyle ou aryle peuvent être obtenus par cyclisation de composés de formule VIII :

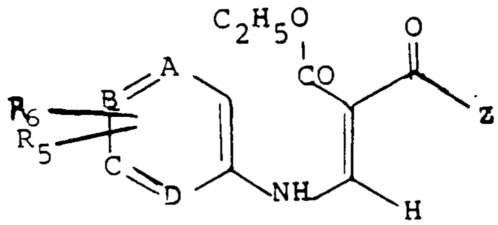

VIII

dans laquelle $R_5$, $R_6$ et A, B, C, D ont les mêmes significations que dans la formule I, et Z représente un groupe alkyle en $C_1$ à $C_4$, ou aryle en $C_4$ à $C_6$ tel que phényle, furyle, thiényle, pyridyle, imidazolyle ou pyrolyle.

On peut effectuer cette cyclisation par chauffage du composé VIII, à une température comprise entre 180°C et 310°C, dans un solvant de point d'ébullition élevé, tel que le diphényle ou le diphényléther ou leur mélange.

La cyclisation peut aussi être réalisée par action de l'acide sulfurique et de l'anhydride acétique selon un procédé décrit par R.K. MAPARA, dans J. Indian Chem. Soc., 1954, 31, 951, ou par action d'un ester polyphosphorique (PPE) comme décrit par H. AGUI, dans J. Heterocyclic. Chem. 1975, 557.

Les composés de formule VIII peuvent être obtenus par réaction d'un mélange équimoléculaire d'orthoformiate d'éthyle, d'amine aromatique et de dérivé d'acétate d'éthyle selon le schéma réactionnel:

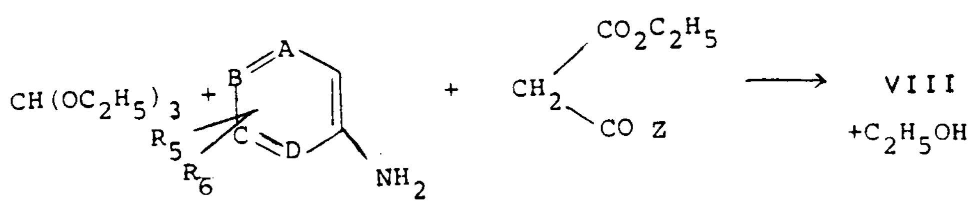


dans ces formules $R_5$, $R_6$, Z et A, B, C, D ont les mêmes significations que dans la formule VIII.

La réaction s'effectue en général à une tem-pérature de 80 à 160°C, de préférence dans un solvant, en éliminant l'éthanol au fur et à mesure de sa formation.

Les composés de formule VIII peuvent aussi être préparés à partir de l'amine aromatique du schéma ci-dessus, sur laquelle on fait réagir l'orthoformiate d'éthyle pour donner, de façon connue, une formamidine de formule :

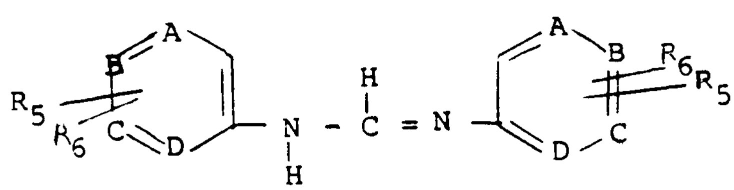

IX

dans laquelle $R_5$, $R_6$ et A, B, C, D ont la même signification que dans la formule I et qui donne le produit de formule VIII par réaction avec le dérivé d'acétate d'éthyle du schéma précédent en présence d'un excès d'orthoformiate d'éthyle.

Les composés de formule III dans lesquels X représente un halogène sont préparés, de façon classique par réaction des dérivés hydroxylés correspondants, avec $SOCl_2$, $POCl_3$ ou $PCl_5$ lorsque X = Cl ou avec $PBr_3$ ou $POBr_3$ lorsque X = Br, de préférence en excès, à une température comprise entre 60°C et 190°C, éventuellement dans un solvant tel que le dichloroéthane, le toluène ou le chlorobenzène.

Les composés de formule I qui sont des N-oxydes peuvent être préparés à partir des N-oxydes des composés de formule III dans laquelle X représente un halogène; ceux-ci résultent de l'action d'un péracide sur l'amine, selon un procédé classique par exemple par action de l'acide m-chloroperbenzoïque sur la quinoléine ou la naphtyridine en solution dans l'acide acétique.

Les composés de formule I dans lesquels Z = OH sont obtenus par action d'une base, par exemple d'un carbonate alcalin en milieu hydroalcoolique sur l'ester correspondant, suivie de la libération de l'acide de son sel.

Les composés de formule I dans lesquels Z = $NR_8R_9$ sont obtenus par action de l'amine $HNR_8R_9$ sur le composé de formule I dans lequel Z = $OR_7$, et de préférence $OC_2H_5$, à une température comprise entre 60 °C et 120 °C sans solvant ou dans un solvant tel que le diméthylformamide. Ils peuvent être obtenus aussi à partir des composés de formule III dans laquelle X est un halogène et Z est Cl, que l'on fait réagir sur $NH_3$ ou l'amine de formule $HNR_8R_9$ pour obtenir le composé de formule III dans lequel Z est $HNR_8R_9$, les composés de formule I étant ensuite préparés de façon classique; certains des chlorures d'acide de départ ont été décrits dans FR-A-2 581 382.

Les énantiomères des composés de formule I peuvent être préparés soit à partir des amines de formule II optiquement actives, notamment lorsque les amino-acides de formule

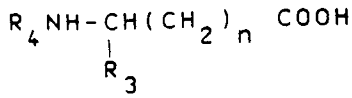

sont commerciaux, soit par recristallisation d'un sel de l'amine de formule I racémique avec un acide optiquement actif tel que les acides camphosulfoniques lévogyre ou dextrogyre, selon une technique connue.

Les sels d'addition des fonctions amines des composés de formule I sont préparés par action d'un acide minéral ou organique sur l'amine en solution et le sel est isolé soit par évaporation du solvant soit par addition d'un non solvant dans le milieu.

Les sels d'acide carboxylique sont préparés par action d'une base minérale telle que NaOH ou d'une base organique sur l'acide en solution.

L'invention concerne aussi les médicaments comprenant au moins un composé de formule I ou l'un de ses sels pharmaceutiquement acceptables.

Ces composés ont, en effet, une affinité, in vitro et in vivo, pour les récepteurs des benzodiazépines de type périphérique sans présenter d'affinité spécifique pour les récepteurs centraux des benzodiazépines.

On sait que les ligands de ces récepteurs périphériques ont une action sur le système cardiovasculaire, comme vasodilatateurs périphériques qui augmentent le flux sanguin coronaire, qu'ils sont immunomodulateurs ou qu'ils peuvent modifier le comportement en étant anxiolytiques.

Les composés selon l'invention pourront être, selon les cas, utilisés chez l'homme pour la prévention et le traitement des maladies cardiovasculaires ou encore comme antiallergiques et dans la prévention et le traitement des états infectieux ou encore pour le traitement des états anxieux. Chez les animaux et notamment les bovins, les porcins, les ovins et les caprins, les composés selon l'invention seront utilisés pour la prévention et le traitement des états infectieux, notamment en renforçant leurs défenses immunitaires.

Les médicaments de l'invention seront administrés chez l'homme par exemple par voie orale, sous forme de comprimés, gélules ou granulés, ou par voie rectale sous forme de suppositoires ou par voie parentérale sous forme de soluté injectable, à raison de 1 mg à 400 mg par jour, en une ou plusieurs prises selon la structure du composé, l'âge du malade et la nature et la gravité de la maladie.

Ils pourront aussi être administrés aux animaux, incorporés à l'alimentation ou par injection, à des doses comprises entre 0,01 mg/jour et 100 mg/kg/jour.

Les composés de formule I, ou leurs sels, seront associés aux excipients usuels compatibles avec leur réactivité chimique, pour donner des formes pharmaceutiques à libération immédiate ou prolongée.

Les exemples suivants illustrent l'invention. Les analyses élémentaires des produits isolés répondent aux normes habituelles. Les produits sont caractérisés par leurs points de fusion instantanés éventuellement par leur spectre RMN (m signifie massif, d doublet, s singulet, t triplet et (x H) que la bande correspond à x protons) la référence interne est $Si(CH_3)_4$); les spectres infra rouge présentent les bandes caractéristiques des structures attendues. Sauf mention contraire p = o dans les exemples.

1. Préparation des amines de formule II :

a) N,N-dipropyl (amino-2) propanamide

(formule II : $R_1 = R_2 = C_3H_7$, $R_3 = CH_3$, $R_4 = H$, n = 0)

58g de N-(terbutoxycarbonyl) alanine, préparée à partir d'alanine racémique, 33g de N-hydroxysuccinimide et 59g de dicyclohexylcarbodiimide, en solution dans 1400 ml de dioxanne, sont maintenus sous agitation pendant 6 heures. On sépare alors le précipité et évapore le solvant sous pression réduite. Le résidu est lavé par une solution aqueuse de $Na_2CO_3$ à 5%.

Après séchage, on isole 81g de composé de formule IV :
($R_3$ = $CH_3$, $R_4$ = H, R = t-$C_4H_9$ , n = 0, $Z_1$ = succinimidoyle) qui fond à 143 °C.
(A partir de L-alanine, on obtient dans les mêmes conditions un produit fondant à 168 °C).

On dissout 80g de ce produit dans 700 ml de tétrahydrofuranne contenant 140 ml de dipropylamine et on laisse 48 heures sous agitation. On évapore alors le solvant sous pression réduite et on obtient après lavage à l'eau et séchage 80g de composé de formule V dans laquelle $R_1$ = $R_2$ = $C_3H_7$, $R_3$ = $CH_3$, $R_4$ = H, R = t-$C_4H_9$, n = 0, qui fond à 92 °C.
(Le produit préparé à partir du produit IV dérivé de L - alanine ne cristallise pas).

Ce composé est dissous dans 400ml de chloroforme: on y ajoute, à 5 °C, 160ml d'acide trifluoracétique en solution dans 200ml de chloroforme et après 3 heures d'agitation, on évapore le solvant sous pression réduite. On verse de l'eau sur le résidu et neutralise par addition de NaOH avant d'extraire le produit attendu dans le dichlorométhane. On obtient ainsi 29g d'huile jaune. Spectre (H-RMN : 60 MHz, DMSOd6)

$\delta$(ppm) : 0,6 - 1,2 (m, 9H); 1,2 - 1,8 (m,4H); 2,2 - 2,6 (s,2H échangeables); 3 - 3,4 (m,5H). (On isole de la même façon l'amine dérivée de la L-alanine ou son trifluoroacétate après évaporation du chloroforme).

b) N,N-dibutyl (amino-2) propanamide :

7g du composé de formule IV, préparé en a) sont dissous dans 100 ml de tétrahydrofuranne contenant 30 ml de N,N-dibutylamine: après 30 heures sous agitation, le solvant est éliminé par distillation et le résidu est lavé à l'eau pour donner 7,5g de produit de formule V, dans laquelle $R_1$ = $R_2$ = $C_4H_9$, $R_3$ = $CH_3$, $R_4$ = H, R = t-$C_4H_9$ et n = 0, qui fond à 80 °C.

Ce produit est dissous dans 100 ml de $CHCl_3$ contenant 28 ml de $CF_3$-COOH à 0 °C: et traité comme son analogue.

c) N-méthyl N-(chloro-4)phényl (amino-2)propanamide :

On introduit lentement, à 5 °C, 17,6ml de chlorure de chloro-2propionyle dans une solution, dans 50ml d'éther éthylique, de 24,2ml de N-méthyl (chloro-4)phénylamine et 30ml de triéthylamine: on laisse le mélange revenir à température ambiante, sous agitation; 5 heures après la fin de l'addition le précipité est séparé, le solvant est évaporé sous pression réduite et le résidu est dissous dans l'éther éthylique; la solution organique est lavée par une solution aqueuse de HCl (0,5 N) puis à l'eau avant élimination du solvant pour donner, avec 55% de rendement, le produit de formule VI, dans laquelle
$R_1$ = $CH_3$ $R_2$ = (Cl-4) $C_6H_4$, $R_3$ = $CH_3$, n = o, X = Cl.

5g de ce composé sont maintenus à 100 °C, pendant 3 heures, dans un autoclave de 100ml contenant 60 ml d'ammoniac liquide. Après 12 heures, au cours desquelles le milieu revient à température ambiante, on élimine l'ammoniac et on extrait l'amine cherchée dans l'éther éthylique.

On obtient ainsi 2,5 g d'huile.

$^1$H RMN (60 mHz, $CDCl_3$)$\delta$: 1,1 - 1,2 (d,6H); 2 - 2,3 (m,2H échangeables); 3,2 (s,3H); 3,3 - 3,6 (m,1H); 6,9 - 7,5 (m,4H).

d) N,N-dipropyl (amino-2)-phényl-3 propanamide (formule II : $R_1$ = $R_2$ = $C_3H_7$, $R_3$ = $CH_2C_6H_5$, $R_4$ = H, n = 0)

26,5g de N-(tert-butoxycarbonyl) phényl alanine (F = 110 °C), préparée par action du ditert-butyldicarbonate sur la phényl alanine racémique, 11,5g de N-hydroxysuccinimide, 20,6g de dicyclohexylcarbodiimide sont introduits dans 500ml de dioxanne; après 4 heures d'agitation, l'insoluble est séparé et le solvant évaporé sous pression réduite. On obtient le produit de formule IV correspondant, fondant à 138 °C.

Après réaction avec 12g de dipropylamine on obtient le produit de formule V correspondant, fondant à 94 °C, qui par action de l'acide trifluoracétique dans le chloroforme, donne le trifluoroacétate de l'amide de formule II recherché, fondant à 108 °C, avec un rendement global de 40%.

e) amino-2 N-méthyl N-(chloro-4 phényl)acétamide (formule II : $R_1$ = $CH_3$, $R_2$ = 4-$ClC_6H_4$, $R_3$ = $R_4$ = H, n = O)

Dans une solution dans la tétrachlorure de carbone, de 111,5g de chlorure d'acide de N-phtaloylglycine, on introduit simultanément goutte à goutte entre 0° et 5 °C, 70,8g de N-méthyl (chloro-4)aniline et 101g de triéthylamine. On élimine le chlorhydrate de triéthylamine précipité en fin de réaction, après lavage 2 fois avec 100 ml de $CCl_4$. Après séchage des solutions organiques et élimination du solvant, on obtient avec 95% de rendement le phtalimide de formule VII de F = 180 °C (isopropanol).

79,5g de ce composé est mis en suspension dans 1500 ml d'une solution 0,16 M de $N_2H_4$, $H_2O$ dans $C_2H_5OH$ et le milieu est maintenu 3 heures au reflux avant distillation du solvant. Le résidu es repris dans 500 ml de HCl aqueux 2N et l'ensemble est maintenu 2 heures à 50°C. Après filtration, le filtrat est amené à sec et le résidu est recristallisé dans l'isopropanol (rdt 75%) F = 186°C.

2. Préparation des quinoléines de formule III

1) Benzoyl-3 dichloro-4,6 quinoléine.

a) Préparation du benzoyl-3 chloro-6 hydroxy-4 quinoléine, (III : $R_5$ = 6-Cl; $R_6$ = H; $R_{10}$ = H; Z = $C_6H_5$; A = B = C = D = CH; X = OH)

Un mélange de chloro-4 aniline (25,5g), d'orthoformiate d'éthyle (33,3 ml) et de benzoylacétate d'éthyle (34,6 ml) est chauffé à 165°C jusqu'à ce que l'alcool formé soit entièrement distillé. Par addition d'éther de pétrole, l'intermédiaire (chloro-4 anilino)-3 benzoyl-2 acrylate d'éthyle cristallise. Il est recristallisé dans le cyclohexane, F = 111°C (Rdt 45%) puis ajouté à 150 ml d'oxyde de diphényle porté à une température de 200°C.

L'ensemble est chauffé à 240°C pendant 5 heures tout en effectuant une distillation lente. Après addition d'éther de pétrole les cristaux obtenus sont filtrés et séchés sous vide F>260°C (Rdt 86%).

b) Préparation du benzoyl-3 dichloro-4,6 quinoléine (III : X = Cl; $R_5$ = 6-Cl; $R_6$ = H; $R_{10}$ = H; Z = $C_6H_5$; A = B = C = D = CH)

2 g de benzoyl-3 chloro-6 hydroxy-4 quinoléine sont ajoutés par petites quantités sous azote à 10 ml de chlorure de phosphoryle. La solution est portée 3 heures au reflux. Après évaporation du solvant, le résidu est versé sur de la glace et le mélange neutralisé par addition de carbonate de sodium. Le produit attendu est extrait au dichlorométhane : cristaux (éther isopropylique) F = 148°C (Rdt 48%).

Les quinoléines de formule III, dans laquelle X = Cl, $R_6$ = H mentionnées dans le tableau I suivant ont été préparées en appliquant le même mode opératoire que ci-dessus.

TABLEAU I

| Z | $R_5$ | F°C |
|---|---|---|
| $C_6H_5$ | 7-F | 95 |
| $C_6H_5$ | 7-$CF_3$ | 97 |
| $C_6H_5$ | 6-Cl | 148 |
| $C_6H_5$ | 7-Cl | 121 |
| $C_6H_5$ | 8-Cl | 171 |
| $C_6H_5$ | 5-Cl | 135 |
| $C_6H_5$ | H | 130 |
| $C_6H_5$ | 6-$OCH_3$ | 147 |
| $C_5H_4N$-4 | 7-Cl | 132 |
| $C_5H_4N$-3 | 7-Cl | 134 |
| $C_6H_5$ | 7-Br | 134 |
| $C_6H_5$ | 6-Br | 140 |

2) Benzoyl-3 chloro-4 méthoxy-6 quinoléine (III : X = Cl, $R_5$ = 6-$OCH_3$, $R_6$ = H; $R_{10}$ = H; Z = $C_6H_5$, A = B = C = D = CH).

a) bis(méthoxy-4 phényl) formamidine

20 g de méthoxy-4 aniline et 166 ml d'orthoformiate d'éthyle sont chauffés 2 heures à 150°C. L'alcool formé est distillé simultanément. On précipite la formamidine par addition d'éther de pétrole dans le milieu refroidi. F = 114°C (Rdt 40%).

b) benzoyl-2 (méthoxy-4 anilino)-3 acrylate d'éthyle (VIII : $R_5$ = 4-$OCH_3$; $R_6$ = H; Z = $C_6H_5$; A = B = C = D = CH)

Un mélange de bis(méthoxy-4 phényl)formamidine (8 g), d'orthoformiate d'éthyle (6,2 ml) et de benzoylacétate d'éthyle (5,4 ml) est chauffé 2 heures à 170°C en distillant l'alcool formé. On poursuit le chauffage après une nouvelle addition de benzoylacétate d'éthyle (5,4 ml) pendant 2 heures.

Le produit attendu est purifié par chromatographie sur colonne en éluant avec un mélange de cyclohexane + d'acétate d'éthyle (6/4) pour donner une huile claire (Rdt 60%).

c) benzoyl-3 hydroxy-4 méthoxy-6 quinoléine, (III : $R_5$ = 6-$OCH_3$; $R_6$ = H; $R_{10}$ = H; Z = $C_6H_5$; A = B = C = D = CH; X = OH)

10 g de benzoyl-2 (méthoxy-4 anilino)-3 acrylate d'éthyle sont ajoutés à 155 ml d'oxyde de diphényle à 220 °C. L'ensemble est maintenu 20 mn à 245 °C pendant que l'éthanol formé est distillé. Par addition d'éther de pétrole à la solution refroidie, le produit attendu cristallise. F = 260 °C (Rdt 50%).

d) 2 g de benzoyl-3 hydroxy-4 méthoxy-6 quinoléine sont ajoutés à 40 ml de chlorure de phosphoryle et la solution est portée 3 heures au reflux. Après concentration le résidu est versé sur de la glace. La solution est neutralisée par addition de carbonate de sodium, le dérivé chloré est extrait dans le dichlorométhane. Cristaux F = 147 °C (Rdt 85%).

3) Dichloro-4,6 (chloro-4 benzoyl)-3 quinoléine (III : $R_5$ = 6-Cl; $R_6$ = H; Z = (Cl-4)$C_6H_4$; A = B = C = D = CH; X = Cl)

a) 2 g de chloro-4 aniline, 2,27 g d'orthoformiate d'éthyle, 3,47 g de chloro-4 benzoyl acétate d'éthyle (préparé selon Burton, J. Chem. Soc. 1928, 904), sont chauffés 4 heures, en distillant l'éthanol. Le (chloro-4 anilino)-3 (chloro-4 benzoyl)-2 acrylate d'éthyle brut obtenu est ajouté ensuite à 76 ml de diphényl éther, le mélange est chauffé à 250 °C pendant 30 minutes. La (chloro-4 benzoyl)-3 chloro-6 hydroxy-4 quinoléine est obtenue avec un rendement de 53%. F> 260 °C.

b) 2,5 g du produit précédent sont ajoutés à 25 ml de chlorure de phosphoryle.

On chauffe 3 heures au reflux. Après traitement la dichloro-4,6 (chloro-4 benzoyl)-3 quinoléine est obtenue, F = 170 °C (Rdt 90%).

4) Dichloro-4,7 isonicotinoyl-3 quinoléine (III : X = Cl; $R_5$ = 7-Cl; $R_6$ = H; $R_{10}$ = H; Z = $C_5H_4N$-2; A = B = C = D = CH)

a) isonicotinoyl-acétate d'éthyle :

l'isonicotinate d'éthyle (20g 0,132 mole) est introduit dans une suspension dans le tétrahydrofuranne (120 ml) d'hydrure de sodium (0,185 mole) et le mélange st porté au reflux. Après addition d'acétate d'éthyle (19,4 ml 0,198 mole) le reflux est maintenu une nuit. Après hydrolyse et évaporation du solvant organique, la phase aqueuse est lavée à l'acétate d'éthyle et elle est acidifiée par addition d'acide acétique. Après extraction par le dichlorométhane, et évaporation du solvant organique, les cristaux obtenus sont séchés sous vide. F = 70 °C (Rdt 86%).

b) (chloro-3 anilino)-3 isonicotinoyl-2 acrylate d'éthyle, (VIII : $R_5$ = 3-Cl; $R_6$ = H; Z = $C_5H_4N$-3; A = B = C = D = CH)

Un mélange de bis(chloro-3 phényl) formamidine (5g), d'isonicotinoyl acétate d'éthyle (3,64g) et d'orthoformiate d'éthyle (3,74 ml) est chauffé 45 minutes à 140 °C, avant une nouvelle addition d'isonicotinoyl acétate d'éthyle (3,64 g); le milieu réactionnel est alors encore chauffé 2 heures à 150 °C.

Le produit attendu est purifié par chromatographie sur colonne de silice, en éluant avec un mélange cyclohexane/acétate d'éthyle (1/1) puis recristallisé dans un mélange d'éther isopropylique/acétate d'éthyle (8/2) F = 93 °C (Rdt 87%).

$^1$H RMN (80 MHZ, $CDCl_3$) 0,8-1,2 (t, 3H) 3,8-4,2 (q, 2H) 7-7,5 (m, 6H) 8,4-8,8 (m, 3H) 12-12,5 (d, 1H échangeable).

c) chloro-7 hydroxy-4 isonicotinoyl-3 quinoléine, (III : $R_5$ = 7-Cl; $R_6$ = H; $R_{10}$ = H; Z = $C_5H_4N$-2; A = B = C = D = CH; X = OH)

5,3 g de (chloro-3 anilino)-3 isonicotinoyl-2 acrylate d'éthyle sont chauffés 20 minutes à 250 °C dans 85 ml de diphényléther. Les cristaux obtenus par refroidissement sont lavés à l'éther de pétrole puis recristallisés dans diméthylacétamide. F > 260 °C (Rdt 60%).

d) dichloro-4,7 isonicotinoyl-3 quinoléine

2,7 g de chloro-7 hydroxy-4 isonicotinoyl-3 quinoléine sont ajoutés sous azote à 60 ml de $POCl_3$ et portés 5 heures au reflux. Après filtration le précipité est versé dans la glace. La solution aqueuse est basifiée par le carbonate de sodium avant d'en extraire dans le dichlorométhane, le dérivé chloré : cristaux (éther isopropylique) F = 132 °C (Rdt 53%).

3. Préparation des naphtyridines de formule III

1) benzoyl-3 chloro-4 naphtyridine-1,5 (III: X = Cl; $R_5$ = $R_6$ = $R_{10}$ = H; Z = $C_6H_5$; A = N; B = C = D = CH)

a) (pyridyl-3)amino-3 benzoyl-2 acrylate d'éthyle, (VIII : $R_5$ = $R_6$ = H; Z = $C_6H_5$; A = N; B = C = D = CH)

Un mélange équimoléculaire d'amino-3 pyridine, d'orthoformiate d'éthyle et de benzoylacétate d'éthyle est chauffé 3 heures à 125-130 °C, sous un léger courant d'azote. La réaction terminée, le produit est purifié par chromatographie sur colonne de silice (toluène-éthanol, 95-5). F = 115 °C (éther isopropylique) Rdt 60%.

1H RMN (60 MHz $CDCl_3$ + acide trifluoroacétique) : 0,7-1,2 (m,3H) 3,8-4,2 (q,2H) 7,1-7,9 (m,7H) 8,3-8,8 (m,3H).

b) benzoyl-3 hydroxy-4-naphtyridine-1,5 (III : $R_5$ = $R_6$ = H; $R_{10}$ = H; Z = $C_6H_5$; A = N; B = C = D = CH; X = OH)

120 g de (pyridyl-3) amino-3 benzoyl-2 acrylate d'éthyle sont cyclisés dans le Dowtherm A (mélange de 816 ml de diphényloxyde et 318 g de biphényle) par chauffage à 245-250 °C, pendant 30 minutes. Le composé qui précipite dans le milieu après refroidissement à température ambiante, est filtré et lavé à l'éther de pétrole Rdt 40%; F>260 °C.

c) benzoyl-3 chloro-4 naphtyridene-1,5 (III : X = Cl; $R_5$ = $R_6$ = H; $R_{10}$ = H; Z = $C_6H_5$; A = N; B = C = D = CH)

19,5 g de benzoyl-3 hydroxy-4 naphtyridine -1,5 sont ajoutés à 200 ml d'oxychlorure de phosphore au reflux. Le reflux est maintenu 0,5 heure. La réaction terminée, l'excès d'oxychlorure de phospore est éliminé sous pression réduite et le résidu huileux est neutralisé par addition de NaOH aqueux concentré. Après extraction par du dichlorométhane, la phase organique est lavée à l'eau puis séchée sur sulfate de magnésium. Le produit est recristallisé dans cyclohexane. F = 119 °C (Rdt 60%).

2)[(bromo-7-chloro-4 naphtyridine-1,5)yl-3] carboxylate d'éthyle

obtenu en applicant le mode opératoire précédent, au dérivé hydroxylé; ce produit fond à 130 °C.

3) benzoyl-3 chloro-4 méthyl-2 naphtyridine-1,5 (formule II : X = Cl; $R_5$ = $R_6$ = H; $R_{10}$ = $CH_3$; Z = $C_6H_5$: A = N; B = C = D = CH; p = O)

a) benzoyl-3 hydroxy-4 méthyl-2 naphtyridine-1,5.

Le benzoyl-2(pyridyl-3)amino-3 crotonate d'éthyle, préparé par condensation de benzoyl-2 éthoxy-3 crotonate d'éthyle et d'amino-3 pyridine, est cyclisé avec 55% de rendement par chauffage dans le Dowtherm selon le procédé décrit précédemment. F > 260 °C.

$^1$HRMN (80MHz, $DMSOd_6$)$\delta$: 2,4 (s,3H); 7,4-8,2 (m,7H); 8,7-8,9 (d,1H); 12,1-12,4(m,1H).

b) 7g du dérivé précédent sont dissous dans 50 ml de toluène et on ajoute, à la température de reflux du solvant, 10 ml de $POCl_3$, goutte à goutte. Le reflux est maintenu pendant 30 minutes après la fin de l'addition et les solvants sont évaporés. Après neutralisation et séchage, le résidu fond à 120 °C. Rendement 40%.

4) benzoyl-3 chloro-4 naphtyridine-1,6 (III : X = Cl; $R_5$ = $R_6$ = H; $R_{10}$ = H; Z = $C_6H_5$, A = C = D = CH, B = N)

28 g de benzoyl-3 hydroxy-4 naphtyridine-1,6, F > 260 °C, préparé à partir de l'amino-4 pyridine en appliquant le procédé décrit précédemment pour la préparation de la benzoyl-3 chloro-4 naphttyridine-1,5 sont additionnés à 300 ml d'oxychlorure de phosphore et le mélange est maintenu une heure au reflux. Après traitement, le composé attendu est recristallisé dans l'acétate d'éthyle. Rdt 80%.

$^1$H RMN (60 MHz, $CDCl_3$) 7,4-8 (m,6H) 8,8-9 (m,2H) 9,75 (s,1H).

5) benzoyl-3 dichloro-4,7 naphtyridene-1,8 (III : X = Cl; $R_5$ = 7-Cl; $R_{10}$ = $R_6$ = H; Z = $C_6H_5$, A = B = C = CH, D = N)

a) benzoyl-3 chloro-7 hydroxy-4 naphtyridine-1,8 (III : $R_5$ = 7-Cl; $R_6$ = $R_{10}$ = H; Z = $C_6H_5$; A = B = C = CH, D = N; X = OH)

Au mélange, porté à 210 °C, de 185 ml de diphényloxyde et 65 g de biphényle est ajouté 27,5 g de (chloro-6 pyridyl-2) amino-3 acrylate d'éthyle. L'addition terminée, on élève la température jusqu'à 240-245 °C et maintenant cette température pendant 2 heures. Après refroidissement le composé qui précipite est filtré et est lavé à l'éther de pétrole. (F = > 260 °C).

$^1$H RMN (80MHz, DMSO-$d_6$)7,4-8 (m,6H) 8,3-8,7 (m,2H) 13-13,5 (m,1H)

b) benzoyl-3 dichloro-4,7 naphtyridine- 1,8:

9g de benzoyl-3-chloro-7 hydroxy-4 naphtyridine-1,8 et 30 ml d'oxychlorure de phosphore sont portés au reflux pendant une heure. L'oxychlorure de phosphore en excès est éliminé par distillation sous pression réduite et le résidu est dissous dans le dichlorométhane. Cette phase organique est lavée jusqu'à neutralité par une solution aqueuse de NaOH. Après décantation et lavage à l'eau, la solution de dichlorométhane est séchée sur sulfate de sodium puis concentrée. Le produit est recristallisé dans le cyclohexane. F = 160 °C (Rdt 66%)

$^1$H-RMN (60 MHz, $CDCl_3$) 7,4-7,9 (m,6H) 8,5-8,7 (d, 1H) 9 (s,1H).

6) [(dichloro-4,7 naphtyridine-1,8)yl-3] carboxylate d'éthyle

10 g de [(chloro-7 hydroxy-4 naphtyridine-1,8)yl-3] carboxylate d'éthyle et 100 ml de $POCl_3$ sont portés au reflux, durant 1 heure, puis l'excés de $POCl_3$ est éliminé par distillation et le résidu, repris avec du chlorure de méthylène est neutralisé par addition d'une solution aqueuse concentrée de NaOH. Après séchage et évaporation du solvant le résidu est purifié par chromatographie sur colonne de silice en éluant avec un mélange de toluène et d'éthanol (98/2 -v/v). Après recristallisation dans le cyclohexane, la naphtyridine fond à 134-136 °C.

7) benzoyl-3 chloro-4 naphtyridine-1,7 (formule III : X = Cl; $R_5$ = $R_6$ = $R_{10}$ = H; Z : $C_6H_5$; A = B = D = CH ; C = N; p = O)

a) 7-oxyde de benzoyl-3 hydroxy-4 naphtyridine-1,7 préparé à partir d'amino-3 pyridine 1-oxyde, décrit par J.C. Murray et C.R. Hauser dans J. Org. Chem. p. 2008-14 (1954), et de benzoylacétate d'éthyle et d'orthoformiate d'éthyle selon le procédé décrit pour la benzoyl-3 hydroxy-4 naphtyridine-1,5; on obtient ce produit avec 70% de rendement F > 260 °C.

b) benzoyl-3 hydroxy-4-naphtyridine-1,7

6,5 g du N-oxyde précédemment préparé sont introduits dans 240 ml d'acide acétique concentré, avec 12,7 ml d'anhydride acétique et 2g de charbon palladié à 10% et le mélange est agité à température ambiante sous atmosphère d'hydrogène, jusqu'à fin d'absorption de $H_2$. Après filtration, le filtrat est amené à sec et le résidu repris dans 300 ml d'eau est neutralisé par addition de NaOH; le précipité formé est isolé; on obtient ainsi avec 85% de rendement le produit attendu qui fond à 228 °C.

c) 2g du dérivé hydroxyle précédent sont chlorés par chauffage dans 20 ml de $POCl_3$ au reflux, pendant 45 minutes; le produit isolé après évaporation de l'excès de $POCl_3$ et neutralisation est purifié par chromatographie sur colonne de silice, en éluant par un mélange de toluène et d'éthanol (99/1); la naphtyridine obtenue avec 60% de rendement fond à 123 °C.

$^1$HRMN (80MHz, $CDCl_3$)$\delta$: 7,4-7,9 (m,5H); 8-8,2 (d,1H); 8,7-8,9 (d,1H); 9 (s,1H); 10,6 (s,1H).

8) benzoyl-3 chloro-4 naphtyridine-1,5 oxyde-1 (formule III : X = Cl; $R_5$ = $R_6$ = $R_{10}$ = H; Z = $C_6H_5$; A = N; B = C = D = CH; p = 1)

On agite 4 jours à température ambiante 5,37g de benzoyl-3 chloro-4 naphtyridine-1,5 et 3,8g d'acide de m-chloroperbenzoïque dans 200 ml d'acide acétique pur. L'acide acétique est alors évaporé et le résidu repris dans 500 ml de chlorure de méthylène; la phase organique est lavée par une solution saturée de bicarbonate de sodium puis à l'eau; après séchage, le solvent est éliminé et le résidu purifié par chromatographie et recristallisé dans le toluène. F = 135 °C.

$^1$HRMN (80 MHz,$CDCl_3$)$\delta$: 7,6-8,3 (m,6H); 9-9,2 (m,2H); 9,3-9,5 (m,1H).

<u>EXEMPLE 1</u> :

[(N,N-dipropylcarbamoyl-1 éthylamino)-4 trifluorométhyl-7 quinoléinyl-3]carboxylate d'éthyle (formule I : $R_1$ = $R_2$ = $C_3H_7$; $R_3$ = $CH_3$, $R_4$ = H, n = p = o, $R_5$ = H, $R_6$ = 7-$CF_3$, $R_{10}$ = H, Z = $OC_2H_5$, A = B = C = D = CH) numéro de référence : SR 26241.

On chauffe 6 heures à sa température de reflux, une solution dans 100ml d'éthanol, de 2,6g de N,N-dipropyl(amino-2) propanamide, 3g de (chloro-4 trifluorométhyl-7 quinoléinyl-3)carboxylate d'éthyle (F = 60 ° C) et 2 ml de triéthylamine.

Après élimination du solvant le résidu est repris dans l'eau et le dichlorométhane. La phase organique est décantée, le solvant évaporé et le résidu purifié par chromatographie sur une colonne de silice en éluant à l'éther isopropylique. Après recristallisation dans l'hexane, on obtient avec 32% de rendement le produit recherché; F = 100 ° C.

$^1$H RMN (80 mHz, $CDCl_3$)δ; 0,6-1 (m,6H); 1,3-1,8 (m,10H); 2,9-3,8 (m,4H); 4,3-4,7 (q,2H); 4,9-5,3 (m,1H); 7,5-8,35 (m,3H); 9,2 (s,1H); 9,5-9,7 (d,1H échangeable).

EXEMPLES 2 à 30 : esters dérivés de quinoléine

Ces exemples de composés de formule I dans laquelle A = B = C = D = CH, et n = p = o figurent dans le tableau II; les produits ont été préparés en appliquant le méthode décrite à l'exemple 1.

TABLEAU II

| EX N° | SR | $R_1$ | $R_2$ | $R_3$ | $R_4$ | $R_5$ | $R_6$ | Z | $R_{10}$ | Caractéristiques physiques F°C(sel) | $[\alpha]^{20}$ (C, SOLVANT) |
|---|---|---|---|---|---|---|---|---|---|---|---|
| 2 | 26399 | $C_3H_7$ | $C_3H_7$ | $CH_3$ | H | H | 5-Cl | $OC_2H_5$ | H | 94 | |
| 3 | 26058 | $C_3H_7$ | $C_3H_7$ | $CH_3$ | H | H | 6-Cl | $OC_2H_5$ | H | 59 | |
| 4 | 26274 | $C_3H_7$ | $C_3H_7$ | $CH_3$ | H | H | 6-Cl | $OC_2H_5$ | H | 170(HCl) | -19(1;$H_2O$) |
| 5 | 26310 | $C_3H_7$ | $C_3H_7$ | $CH_3$ | H | H | 7-Cl | $OC_2H_5$ | H | 83 | |
| 6 | 26378 | $C_3H_7$ | $C_3H_7$ | $CH_3$ | H | H | 7-Cl | $OC_2H_5$ | H | 160(HCl) | -0,9(1; $CH_3OH$) |
| 7 | 26422 | $C_3H_7$ | $C_3H_7$ | $CH_3$ | H | H | 7-Cl | $OC_2H_5$ | H | 175(HCl) | +1,9(1; $CH_3OH$) |
| 8 | 26351 | $C_3H_7$ | $C_3H_7$ | $CH_3$ | H | H | 8-Cl | $OC_2H_5$ | H | 98 | |
| 9 | 26377 | $C_3H_7$ | $C_3H_7$ | $CH_3$ | H | H | 7-Br | $OC_2H_5$ | H | 160(HCl,H2O) | |
| 10 | 26421 | $C_3H_7$ | $C_3H_7$ | $CH_3$ | H | 6-$CH_3$ | 7-$CH_3$ | $OC_2H_5$ | H | 170(HCl,H2O) | |
| 11 | 26492 | $C_3H_7$ | $C_3H_7$ | $CH_3$ | H | 6-Cl | 7-Cl | $OC_2H_5$ | H | 106 | |
| 12 | 26357 | $C_3H_7$ | $C_3H_7$ | $CH_3$ | H | H | 6-$OCH_3$ | $OC_2H_5$ | H | 140(maléate) | |
| 13 | 26522 | $C_3H_7$ | $C_3H_7$ | $CH_3$ | H | H | 7-$OCH_3$ | $OC_2H_5$ | H | 102 | |
| 14 | 25552 | $C_3H_7$ | $C_3H_7$ | $CH_3$ | H | | 6,7-$OCH_2O$ | $OC_2H_5$ | H | 130(maléate, $H_2O$) | |
| 15 | 26426 | $C_3H_7$ | $C_3H_7$ | $CH_3$ | H | H | H | $OC_2H_5$ | H | 170(maléate) | |
| 16 | 26487 | $C_3H_7$ | $C_3H_7$ | $CH_3$ | H | H | 6-cyclo-hexyl | $OC_2H_5$ | H | 100 | |
| 17 | 26449 | $C_3H_7$ | $C_3H_7$ | $CH_3$ | H | H | 7-Cl | $OC_4H_9$ | H | 84 | |
| 18 | 26454 | $C_2H_5$ | $C_2H_5$ | $CH_3$ | H | H | 7-Cl | $OC_2H_5$ | H | 100 | |
| 19 | 26362 | $C_4H_9$ | $C_4H_9$ | $CH_3$ | H | H | 7-$CF_3$ | $OC_2H_5$ | H | 140 | |
| 20 | 26423 | $CH_3$ | (4-Cl)-$C_6H_4$ | $CH_3$ | H | H | 7-Cl | $OC_2H_5$ | H | 146 | |

TABLEAU II (suite)

| EX N° | SR | $R_1$ | $R_2$ | $R_3$ | $R_4$ | $R_5$ | $R_6$ | Z | $R_{10}$ | Caractéristiques physiques F°C(sel) $[\alpha]^{20}$ (C, SOLVANT) |
|---|---|---|---|---|---|---|---|---|---|---|
| 21 | 26306 | $C_3H_7$ | $C_3H_7$ | $C_2H_5$ | H | H | 7-$CF_3$ | $OC_2H_5$ | H | 60 |
| 22 | 26258 | $C_3H_7$ | $C_3H_7$ | $CH_2C_6H_5$ | H | H | 7-$CF_3$ | $OC_2H_5$ | H | 95 |
| 23 | 26319 | $C_3H_7$ | $C_3H_7$ | $CH_3$ | H | H | 6-Cl | $OC_2H_5$ | $CH_3$ | huile |
| 24 | 26269 | $C_3H_7$ | $C_3H_7$ | $CH_3$ | H | H | 6-Cl | $OC_2H_5$ | $C_6H_5$ | 110 |
| 25 | 26641 | $C_3H_7$ | $C_3H_7$ | $CH_3$ | H | 6-$OCH_3$ | 7-$OCH_3$ | $OC_2H_5$ | H | 160(maléate, $H_2O$) |
| 26 | 26920 | $C_3H_7$ | $C_3H_7$ | $CH_3$ | H | H | 7-F | $OC_2H_5$ | H | 88 |
| 27 | 26619 | $C_3H_7$ | $C_3H_7$ | $CH_3$ | $CH_3$ | H | 7-Cl | $OC_2H_5$ | H | huile |
| 28 | 26632 | $C_3H_7$ | $C_3H_7$ | $CH_3$ | H | H | 7-Cl | OH | H | >260 |
| 29 | 26275 | $C_3H_7$ | $C_3H_7$ | $C_6H_5$ | H | H | 7-$CF_3$ | $OC_2H_5$ | H | 126 |
| 30 | 26529 | $C_3H_7$ | $C_3H_7$ | $C_6H_5$ | H | H | 6-Cl | $OC_2H_5$ | H | 102 |

EXEMPLE 31 :

{[Chloro-6 (N,N-dipropylcarbamoyl-1 éthylamino)-4 naphtyridine-1,5]yl-3} carboxylate d'éthyle. (formule I : $R_1 = R_2 = C_3H_7$, $R_3 = CH_3$, $R_4 = R_5 = H$, $R_6$ = 6-Cl, $R_{10}$ = H, Z = $OC_2H_5$; A = N, B = C = D = CH n = p = o) Référence : SR 26293

On maintient 1 heure, à sa température de reflux, une solution dans 30 ml d'éthanol de 2,7 g de [-(dichloro-4,6 naphtyridine-1,5)yl-3] carboxylate d'éthyle F = 114°C, 2,2 g d'hémisulfate de N,N-dipropyl-(amino-2)propanamideet 3,1 ml de triéthylamine.

Le solvant est alors éliminé sous pression réduite et le résidu est dissous dans 50ml de dichlorométhane; la solution organique est lavée par 2 fois 10ml d'eau, puis le solvant est éliminé, après séchage.

Le résidu est purifié par chromatographie sur colonne de silice en éluant avec un mélange de toluène et d'éthanol (99/1).

Après recristallisation dans le cyclohexane, le produit attendu fond à 133°C.

$^1$H RMN (60MH, $CDCl_3$)$\delta$: 0,6-1 (t,6H); 1,1-1,8 (m,10H); 2,8-3,8 (m,4H); 4,1-4,6 (q,2H); 6,1-6,5 (m,1H); 7,2-7,5 (d,1H); 7,7-8 (s,1H); 8,95 (s,1H); 10,4-10,7 (d,1H échangeable).

EXEMPLE 32 :

{[chloro-6(N,N-dipropylcarbamoylméthyl-1 éthylamino)- 4 naphtyridine-1,5]yl-3} carboxylate d'éthyle (formule I : $R_1 = R_2 = C_3H_7$, $R_3 = CH_3$; $R_4 = R_5 = H$; $R_6$ = 6-Cl; $R_{10}$ = H; Z = $OC_2H_5$; A = N, B = C = D = CH; n = 1; p = o. Référence : SR 26331.

En appliquant le mode opératoire décrit à l'exemple 31, on obtient avec 37% de rendement, le produit recherché qui fond à 102°C après recristallisation dans l'acétate d'éthyle.

$^1$H RMN (80 MHz, $CDCl_3$)$\delta$: 0,55-1,00 (t,6H); 1,15-1,80 (m,10H);2,50-2,95 (m,2H); 3,00-3,50 (m,4H); 4,10-4,50 (q,2H); 5,40-5,80 (m,1H); 7,30-7,50 (d,1H); 7,90-8,10 (d,1H); 9,01 (s,1H); 9,40-9,60 (d,1H).

EXEMPLE 33 :

{[bromo-7(N,N-dipropylcarbamoyl-1 éthylamino)-4 naphtyridine-1,5]yl-3} carboxylate d'éthyle (formule I : $R_1 = R_2 = C_3H_7$, $R_3 = CH_3$, $R_4 = R_5 = R_{10}$ = H, $R_6$ = 7-Br, Z= $OC_2H_5$, n = p = O, A = N, B = C = D = CH). Référence : SR 26579.

3,01g de [(bromo-7 chloro-4 naphtyridine-1,5)yl-3]carboxylate d'éthyle, 2,86g de trifluoracétate de N,N-dipropyl (amino-2) propanamide et 2,7 ml de triéthylamine sont dissous dans 50ml d'éthanol et la solution est portée une heure au reflux. Le solvant est évaporé; une solution du résidu dans le chlorure de méthylène est lavée à l'eau et le solvant évaporé.

Le produit attendue, après recristallisation dans le cyclohexane fond à 96 ° C.

EXEMPLE 34 :

{[chloro-7 (N,N dipropylcarbamoylméthyl-1 éthylamino)-4 naphtyridine-1,5]yl-3} carboxylate d'éthyle (formule I : $R_1$ = $R_2$ = $C_3H_7$; $R_3$ = $CH_3$; $R_4$ = $R_5$ = $R_{10}$ = H; $R_6$ = 7-Cl; Z - $OC_2H_5$; A = N, B = C = D = CH; n = 1; p = O). Référence SR 26869.

En appliquant le mode opératoire décrit à l'exemple 31, on obtient avec 40% de rendement, cet ester qui fond à 88 ° C après recristallisaion dans le pentane.

EXEMPLE 35 :

{[chloro-7(N,N-dipropylcarbamoyl-1 éthylamino)-4 naphtyridine-1 8]yl-3} carboxylate d'éthyle (formule I : $R_1$ = $R_2$ = $C_3H_7$, $R_3$ = $CH_3$, $R_4$ = $R_5$ = $R_{10}$ = H, $R_6$ = 7-Cl, Z = $OC_2H_5$, A = B = C = CH, D = N, n = p = O). Référence : SR 26493.

3g de [(dichloro-4,7 naphtyridine-1,8)yl-3] carboxylate d'éthyle, 2,5g d'hémisulfate de N,N-dipropyl (amino-2)propanamide et 3,6ml de triéthylamine sont introduits dans 30ml d'isopropanol et le mélange est maintenu 3h au reflux. Le solvant est évaporé: le résidu, en solution dans le chlorure de méthylène, est lavé à l'eau puis chromatographié sur colonne de silice en éluant avec un mélange de cyclohexane et d'acétate d'éthyle (80/20). Après recristallisation dans l'éther éthylique, il fond à 93 ° C.

EXEMPLE 36 :

{[chloro-7 (N-méthyl N-(chloro-4 phényl)carbamoyl-l éthylamino)-4 naphtyridine-1,8]yl-3} carboxylate d'éthyle
(formule I : $R_1$ = $CH_3$; $R_2$ = 4-$ClC_6H_4$; $R_3$ = $CH_3$; $R_4$ = $R_5$ = $R_{10}$ = H; $R_6$ = 7-Cl, Z = $OC_2H_5$; A = B = C = CH, D = N; n = p = o). Référence SR 26555.

Préparé avec un rendement de 10% environ, en appliquant le mode opératoire décrit à l'exemple 35, ce produit après recristalisation dans l'éthanol fond à 206-208 ° C.

EXEMPLE 37 :

N,N-dipropyl[(benzoyl-3 chloro-6)quinolyl-4]aminoacétamide (formule I : $R_1$ = $R_2$ = $C_3H_7$; $R_3$ = $R_4$ = $R_6$ = $R_{10}$ = H; $R_5$ = 6-Cl; Z = $C_6H_5$; n = p = o; A = B = C = D = CH). Référence SR 26199.

2 g de benzoyl-3 dichloro-4,6 quinoléine et 1,55 g de chlorhydrate d'amino-2 N,N-dipropyl acétamide, préparé selon Haworth et al., J. Chem. Soc., 1952, 2972, sont chauffés au reflux 4 heures en présence de 2,2 ml de triéthylamine dans 50 ml d'isopropanol. Après concentration à sec, l'huile résiduelle est dissoute dans le dichlorméthane, et la phase organique est lavée à l'eau.

Le produit attendu peut être purifié par chromatographie sur gel de silice (éluant : cyclohexane-acétate d'éthyle (1/1) : on obtient des cristaux jaune clair. F = 109 ° C - 110 ° C (Rdt 46%).

$^1$H RMN (80 MHz $Me_2SO$-$d_6$) (p.p.m.) : 0,6-0,8 (t,6H) 1,3-1,7 (m,4H) 2,9-3,3 (m,4H) 4,1-4,3 (d,2H) 7,4-7,9 (m,8H) 8,4 (s,1H) 8,5 (t, 1H, échangeable $D_2O$).

EXEMPLES 38 à 73

Les composés préparés à partir des quinoléines de formule III dans lesquelles Z représente un groupe aryle et $R_6$ = $R_{10}$ = H, n = p = O, en appliquant le procédé décrit à l'exemple 37, sont indiqués dans le tableau III suivant:

TABLEAU III

| EX | REF. SR | $R_1$ | $R_2$ | $R_3$ | $R_4$ | $R_5$ | Z | F°C (sel) |
|---|---|---|---|---|---|---|---|---|
| 38 | 26290 | $C_3H_7$ | $C_3H_7$ | H | H | 7-Cl | $C_6H_5$ | 118 |
| 39 | 26307 | $C_3H_7$ | $C_3H_7$ | H | H | 8-Cl | $C_6H_5$ | 97 |
| 40 | 26303 | $C_3H_7$ | $C_3H_7$ | H | H | H | $C_6H_5$ | 93 |
| 41 | 26372 | $C_3H_7$ | $C_3H_7$ | H | H | $6-OCH_3$ | $C_6H_5$ | 126 |
| 42 | 26483 | $C_2H_5$ | $C_2H_5$ | H | H | 6-Cl | $C_6H_5$ | 108 |
| 43 | 26485 | $C_2H_5$ | $C_2H_5$ | H | H | 7-Cl | $C_6H_5$ | 172 (malé-ate) |
| 44 | 26386 | $C_4H_9$ | $C_4H_9$ | H | H | 7-Cl | $C_6H_5$ | 106 |
| 45 | 26467 | $CH_3$ | $CH(CH_3)C_2H_5$ | H | H | 7-Cl | $C_6H_5$ | 168 (malé-ate) |
| 46 | 26412 | $CH_3$ | $(Cl-4)C_6H_4$ | H | H | 7-Cl | $C_6H_5$ | 126 |
| 47 | 26397 | $C_3H_7$ | $(Cl-4)C_6H_4$ | H | H | 7-Cl | $C_6H_5$ | 181 |
| 48 | 26226 | $C_3H_7$ | $C_3H_7$ | $CH_3$ | H | 6-Cl | $C_6H_5$ | 120 (HCl, $1/2H_2O$ |
| 49 | 26385 | $C_3H_7$ | $C_3H_7$ | $CH_3$ | H | 7-Cl | $C_6H_5$ | 206 (HCl) |
| 50 | 26450 | $CH_3$ | $(Cl-4)C_6H_4$ | $CH_3$ | H | 7-Cl | $C_6H_5$ | 155 |
| 51 | 26294 | $C_3H_7$ | $C_3H_7$ | H | $CH_3$ | 6-Cl | $C_6H_5$ | 117 |
| 52 | 26304 | $C_3H_7$ | $C_3H_7$ | H | H | 6-Cl | $(Cl-4)-C_6H_4$ | 139 |
| 53 | 26439 | $C_3H_7$ | $C_3H_7$ | H | H | 7-Cl | $C_5H_4N-4$ | 162 |
| 54 | 26588 | $CH_3$ | $(Cl-4)C_6H_4$ | H | H | 7-Cl | $C_5H_4N-4$ | 198 |
| 55 | 26610 | $C_3H_7$ | $C_3H_7$ | H | H | 7-Cl | $C_5H_4N-3$ | 170 (malé-ate) |

TABLEAU III (suite)

| EX | REF. SR | $R_1$ | $R_2$ | $R_3$ | $R_4$ | $R_5$ | Z | F°C (sel) |
|---|---|---|---|---|---|---|---|---|
| 56 | 26643 | $CH_3$ | $(Cl-4)C_6H_4$ | H | H | 7-Cl | $C_5H_4N$-3 | 105 |
| 57 | 26581 | $C_3H_7$ | $C_3H_7$ | H | H | 5-Cl | $C_6H_5$ | 217 |
| 58 | 26620 | $CH_3$ | $(Cl-4)C_6H_4$ | H | H | 6-Cl | $C_6H_5$ | 187 |
| 59 | 26644 | $C_3H_7$ | $C_3H_7$ | H | H | 6-Br | $C_6H_5$ | 104 |
| 60 | 26676 | $CH_3$ | (diCl-3,4) $C_6H_4$ | H | H | 7-Cl | $C_6H_5$ | 126 |
| 61 | 26706 | $CH_3$ | $(Cl-4)C_6H_4$ | H | H | 7-F | $C_6H_5$ | 206 |
| 62 | 26778 | $CH_3$ | $(CF_3-4)C_6H_4$ | H | H | 7-Cl | $C_6H_5$ | 204 |
| 63 | 26794 | $CH_3$ | $(Cl-4)C_6H_4$ | H | H | 7-$CF_3$ | $C_6H_5$ | 160 |
| 64 | 26841 | $CH_3$ | $(Cl-4)C_6H_4$ | H | $CH_3$ | 7-Cl | $C_6H_5$ | 145 |
| 65 | 26846 | $CH_3$ | $(OCH_3-4)$ $C_6H_4$ | H | H | 7-Cl | $C_6H_5$ | 111 |
| 66 | 26876 | $CH_3$ | $(Cl-4)C_6H_4$ | H | H | 7-Br | $C_6H_5$ | 125 |
| 67 | 27195 | $CH_3$ | $(OCH_3-4)$ $C_6H_4$ | H | H | 7-Br | $C_6H_5$ | 144 |
| 69 | 27271 | $CH_3$ | $(OC_2H_5-4)$ $C_6H_4$ | H | H | 7-Cl | $C_6H_5$ | 166 |
| 70 | 27778 | $CH_3$ | $(Cl-4)C_6H_4$ | H | H | 7-$CH_3$ | $C_6H_5$ | 110 |
| 71 | 27295 | $CH_3$ | $(SCH_3-4)C_6H_4$ | H | H | 7-Cl | $C_6H_5$ | 140 |
| 72 | 27307 | $CH_3$ | $(OCH_3-2,4)C_6H_3$ | H | H | 7-Cl | $C_6H_5$ | 170 |
| 73 | 26680 | $C_3H_7$ | $C_3H_7$ | $C_6H_5$ | H | 7-Cl | $C_6H_5$ | 165 |

<u>EXEMPLE 74</u> : [(N,N-dipropylcarbamoyl-1 éthylamino)-4 chloro-6 quinoléinyl]-3 carboxamide (Formule I : $R_1 = R_2 = C_3H_7$; $R_3 = CH_3$; $R_4 = R_5 = H$; n = p = O; $R_6$ = 6-Cl, $R_{10}$ = H, Z = $NH_2$, A = B = C = D = CH) numéro de référence 27277 (hydrate)

a) (dichloro-4,6 quinoléinyl)-3 carboxamide

3g de chlorure d'acide (dichloro-4,6 quinoléinyl)-3 carboxylique préparé selon la méthode décrite dans J. Med. Chem. <u>14</u> (1), p. 17-23, (1971) sont mis en solution dans 80 ml de dioxanne dans lequel on fait barboter pendant 2 h. un courant d'ammoniac gazeux à la température du laboratoire. On filtre le précipitéde chlorure d'ammonium puis on évapore le solvant. Le résidu est dissous dans du chlorure de méthylène et la phase organique est lavée à l'eau jusqu'à neutralité puis séchée. Le cristaux obtenus

après évaporation du solvant sont lavés avec de l'éther isopropylique. F = 209 ° C Rdt = 65%.

b) On porte au reflux pendant 3 h 2g de l'amide précédent, 3,4g d'amino-2 N,N-dipropylpropanamide et 3,5 ml de triéthylamine dans 50 ml d'isopropanol. On évapore le solvant, dissout le résidu dans le chlorure de méthylène et lave la phase organique dans l'eau. Après séchage et élimination de $CH_2Cl_2$, le résidu est recristallisé deux fois dans l'acétate d'éthyle. F = 184 ° C (monohydrate) Rdt = 40%.

EXEMPLE 75 : N,N-dipropyl {[(benzoyl-3) naphtyridine-1, 5]yl-4}aminoacétamide (Formule I : $R_1$ = $R_2$ = $C_3H_7$; $R_3$ = $R_4$ = $R_5$ = $R_6$ = $R_{10}$ = H; n = p = o; Z = $C_6H_5$; A = N; B = C = D = CH). Référence SR 26278 (hydrate)

Une solution, dans 30 ml d'éthanol, de 1,075 g de benzoyl-3 chloro-4 naphtyridine-1,5 et 0,86 g de chlorhydrate de l'amino-2 N,N-dipropyl-acétamide et 1,23 ml de triéthylamine est portée au reflux pendant 1,5 heures. Après concentration à sec, le résidu est dissous dans le dichlorométhane et la phase organique est lavée à l'eau avant d'être décantée et séchée sur sulfate de magnésium.

Le composé recherché est purifié par chromatographie sur colonne de silice en éluant avec un mélange toluène/éthanol (99-1). F = 108 ° C (éther diisopropylique) (Rdt 45%).

$^1$H RMN (60 MHz $Me_2$ SO-$d_6$) 0,5-0,9 (m,6H) 1,1-1,7 (m,4H) 2,8-3,3 (q,4H) 3,6 (s,1H (hydrate) 4,2-4,3 (d,2H) 7,4-7,9 (m,6H) 8,1-8,5 (m,2H) 8,7-8,9 (d,-1H) 9,3-9,8 (m,1H échangeable).

EXEMPLE 76 :

N-méthyl N-(chloro-4 phényl) (benzoyl-3 naphtyridine-1,5)yl-4 aminoacétamide 1-oxyde (formule I : $R_1$ = $CH_3$; $R_2$ = Cl-4 $C_6H_4$; $R_3$ = $R_4$ = $R_5$ = $R_6$ = $R_{10}$ = H; n = o; p = 1; Z = $C_6H_5$; A = N; B = C = D = CH). Référence SR 26838.

Un mélange dans 60 ml d'éthanol de 1g de benzoyl-3 chloro-4 naphtyridine-1,5 1-oxyde, 0,9 g de chlorhydrate d'amino-2 N-méthyl N-(chloro-4 phényl) acétamide et de 0,54 ml de triéthylamine est maintenu sous agitation 15 heures à température ambiante; le milieu est concentré à sec puis repris dans le chlo-rure de méthylène. Le produit final est purifié par chromatographie sur colonne de silice en éluant par un mélange de toluène et éthanol (90/10 v/v) et recristallisé dans le toluène. F = 243 ° C. Rendement 40%.

EXEMPLES 77 à 117 :

Les naphtyridines figurant dans le tableau IV pour lesquelles $R_6$ = $R_{10}$ = H, p = o suivant ont été préparées en appliquant le procédé décrit dans l'exemple 75.

TABLEAU IV

| Ex | Réf.SR | Posit.N | $R_1$ | $R_2$ | $R_3$ | $R_4$ | $R_5$ | Z | n | F°C | solvant de recristallisation |
|---|---|---|---|---|---|---|---|---|---|---|---|
| 78 | 26286 | A = N | $C_3H_7$ | $C_3H_7$ | H | H | 6-Cl | $C_6H_5$ | 0 | 128 | acétate d'éthyle |
| 79 | 26494 | A = N | $C_3H_7$ | $C_3H_7$ | H | H | 7-Br | $C_6H_5$ | 0 | 114 | Cyclohexane |
| 80 | 26410 | B = N | $C_3H_7$ | $C_3H_7$ | H | H | H | $C_6H_5$ | 0 | 148 | toluène |
| 81 | 26332 | A = N | $C_5H_{11}$ | $C_5H_{11}$ | H | H | H | $C_6H_5$ | 0 | 88 | acétate d'éthyle |
| 82 | 26528 | A = N | $CH_3$ | $CH(CH_3)C_2H_5$ | H | H | H | $C_6H_5$ | 0 | 134 | toluène |
| 83 | 26361 | A = N | H | $C(C_3H_7)_3$ | H | H | H | $C_6H_5$ | 0 | 186 | toluène |
| 84 | 26276 | A = N | $CH_3$ | $(Cl-4)C_6H_4$ | H | H | H | $C_6H_5$ | 0 | 184 | acétonitrile |
| 85 | 26336 | A = N | $CH_3$ | $(Cl-4)C_6H_4$ | H | H | 6-Cl | $C_6H_5$ | 0 | 208 | acétate d'éthyle |
| 86 | 26516 | A = N | $CH_3$ | $(Cl-4)C_6H_4$ | H | H | 7-Br | $C_6H_5$ | 0 | 204 | toluène |
| 87 | 26409 | B = N | $CH_3$ | $(Cl-4)C_6H_4$ | H | H | H | $C_6H_5$ | 0 | 217 | toluène |
| 88 | 26411 | A = N | $C_3H_7$ | $(Cl-4)C_6H_4$ | H | H | H | $C_6H_5$ | 0 | 173 | |
| 89 | 26359 | A = N | $C_3H_7$ | $C_3H_7$ | $CH_3$ | H | H | $C_6H_5$ | 1 | 115 | cyclohexane |
| 90 | 26455 | A = N | $CH_3$ | $(Cl-4)C_6H_4$ | $CH_3$ | H | H | $C_6H_5$ | 0 | 189 | toluène |
| 91 | 26517 | D = N | $CH_3$ | $(Cl-4)C_6H_4$ | $CH_3$ | H | 7-Cl | $C_6H_5$ | 0 | 192-94 | éthanol |
| 92 | 26554 | D = N | $CH_3$ | $(Cl-4)C_6H_4$ | H | H | 7-Cl | $C_6H_5$ | 0 | 209 | éthanol |
| 93 | 26360 | A = N | $C_3H_7$ | $C_3H_7$ | H | $CH_3$ | H | $C_6H_5$ | 0 | 146 | acétate d'éthyle |
| 94 | 27303 | A = N | $C_2H_5$ | $(F-2Cl-4)C_6H_3$ | H | H | H | $C_6H_5$ | 0 | 155 | cyclohexane |
| 95 | 26871 | A = N | H | $(Cl-4)C_6H_4$ | H | H | H | $C_6H_5$ | 0 | 210 | toluène |
| 96 | 26858 | A = N | $C_2H_5$ | $(Cl-4)C_6H_4$ | H | H | H | $C_6H_5$ | 0 | 160 | éthanol |
| 97 | 26919 | C = N | $CH_3$ | $(Cl-4)C_6H_4$ | H | H | H | $C_6H_5$ | 0 | 165 | toluène |
| 98 | 27297 | A = N | $CH_3$ | $(Cl-4)C_6H_4$ | H | H | H | $(OH-4)C_6H_4$ | 0 | 182 | acétonitrile |
| 99 | 26830 | A = N | $CH_3$ | $(Cl-4)C_6H_4$ | H | H | 7-Cl | $C_6H_5$ | 0 | 195 | toluène |

TABLEAU IV (suite)

| Ex. | Réf.SR | Posit. N | $R_1$ | $R_2$ | $R_3$ | $R_4$ | $R_5$ | Z | n | F°C | solvant de recristallisation |
|---|---|---|---|---|---|---|---|---|---|---|---|
| 100 | 26698 | A = N | $CH_3$ | $(Cl-4)C_6H_4$ | H | H | $6-OCH_3$ | $C_6H_5$ | 0 | 247 | toluène |
| 101 | 26640 | D = N | $CH_3$ | $(Cl-2)C_6H_4$ | H | H | 7-Cl | $C_6H_5$ | 0 | 120 | cyclohexane |
| 102 | 26651 | A = N | $CH_3$ | $(Cl-2)C_6H_4$ | H | H | H | $C_6H_5$ | 0 | 132 | cyclohexane |
| 103 | 26847 | A = N | $CH_3$ | $(CF_3-4)C_6H_4$ | H | H | H | $C_6H_5$ | 0 | 220 | toluène |
| 104 | 27161 | A = N | $CH_3$ | $(CH_3O-4)C_6H_4$ | H | H | H | $C_6H_5$ | 0 | 150 | cyclohexane |
| 105 | 26980 | A = N | $CH_3$ | $(CH_3-4)C_6H_4$ | H | H | H | $C_6H_5$ | 0 | 175 | cyclohexane |
| 106 | 26803 | A = N | $CH_3$ | $C_6H_5$ | H | H | H | $C_6H_5$ | 0 | 105 | cyclohexane |
| 107 | 26731 | A = N | $CH_3$ | $(Cl-4)C_6H_4$ | H | H | H | $C_6H_5$ | 1 | 165 | toluène |
| 108 | 26710 | A = N | $CH_3$ | $(Cl-4)C_6H_4$ | H | $CH_3$ | H | $C_6H_5$ | 0 | 90 | éther de pétrole |
| 109 | 26578 | D = N | $CH_3$ | $(Cl-4)C_6H_4$ | H | $CH_3$ | 7-Cl | $C_6H_5$ | 0 | 198 | éthanol |
| 110 | 27181 | A = N | $CH_2-CH=HC_2$ | $(Cl-4)C_6H_4$ | H | H | H | $C_6H_5$ | 0 | 160 | cyclohexane |
| 111 | 27153 | A = N | $CH_3$ | $(Cl-4)C_6H_4$ | H | H | H | $(CH_3O-4)C_6H_4$ | 0 | 165 | benzène |
| 112 | 26918 | A = N | $CH_3$ | $(Cl-4)C_6H_4$ | H | H | 6,7-Cl | $C_6H_5$ | 0 | 163 | éthanol |
| 113 | 26708 | A = N | $CH_3$ | $(Cl-3,4)C_6H_3$ | H | H | H | $C_6H_5$ | 0 | 176 | cyclohexane |
| 114 | 26972 | A = N | $CH_3$ | $(Cl-2,4)C_6H_3$ | H | H | H | $C_6H_5$ | 0 | 154 | cyclohexane |
| 115 | 26947 | A = N | $CH_3$ | $(Cl-2,6)C_6H_3$ | H | H | H | $C_6H_5$ | 0 | 195 | éthanol |
| 116 | 27249 | A = N | $CH_3$ | cyclohexyl | H | H | H | $C_6H_5$ | 0 | 185 | éthanol |
| 117 | 27252 | A = N | $CH_3$ | $(F-2Cl-4)C_6H_3$ | H | H | H | $C_6H_5$ | 0 | 138 | cyclohexane |

Les composés des exemples précédents sont peu toxiques, et chez la souris leur DL50 par voie orale est en général supérieure à 1000 mg/kg; ainsi celle du composé de l'exemple 1 est de 1100 mg/kg et celle de l'exemple 84 est supérieure à 1800 mg/kg. Les produits des exemples 3, 67, 90, 107, 114 sont atoxiques à la dose de 500 mg/kg p.o. chez la souris.

On a aussi étudié leur affinité in vitro, pour les récepteurs centraux des benzodiazépines et ceux de type périphérique selon les méthodes décrites dans ce qui suit : on a déterminé in vitro la CI50, c'est-à-dire la concentration du produit étudié dans le milieu qui inhibe 50% de la fixation sur les récepteurs des benzodiazépines présents, d'un ligand connu comme spécifique de ces récepteurs. Les résultats obtenus montrent que les produits de l'invention ont une affinité spécifique pour les récepteurs de type périphérique mais ne se fixent pas sur les récepteurs centraux.

Les méthodes mises en oeuvre ont été les suivantes :

a) détermination de la concentration du produit étudié inhibant 50% de la fixation spécifique sur les récepteurs de type périphérique du (chloro-2 phényl)-1N-méthyl N-(méthylpropyl)isoquinolyl-3 carboxamide (ou PK 11195), ligand connu de ces récepteurs.

La technique est analogue à celle décrite par J. Benavides et Coll. dans Brain Res. Bull. 13 p. 69-77 (1984) et références citées.

Les suspensions de protéines membranaires de cerveau de chat ont été préparées comme décrit par J. Benavides.

Les essais d'inhibition de la fixation du $[^3H]$PK11195 sur les récepteurs par les produits à tester ont été effectués à 4°C, sur 2ml de suspension contenant 0,05mg de protéines et 0,5nM de$[^3H]$PK11195; les incubations ont duré 2h 30.

La fixation non spécifique a été définie comme la quantité de $[^3H]$ PK 11195 déplacé du récepteur par 10 $\mu$M de chloro-7 dihydro-1,3 méthyl-1 (chloro-4 phényl)-5 2H-benzodiazépine-1,4 one-2 ou RO 5-48-64 autre ligand connu.

Les concentrations de produit à tester inhibant 50% de la fixation spécifique de PK11195 au récepteur (CI50), ont été calculées en appliquant la méthode logit-log décrite par Finney dans Probit

analysis-Cambridge University Press. 1979.

b) détermination de la concentration du produit à étudier inhibant 50% de la fixation spécifique du flunitrazépam sur les récepteurs centraux.

Les cerveaux de rats, prélevés après décapitation, sont broyés puis mis en suspension dans une solution aqueuse de saccharose (0,32 M). Le mélange est centrifugé et le culot remis en suspension homo-gène dans une solution tamponnée par TRIS-HCl (50mM; PH = 7,4). Des parties aliquotes de cette suspension sont incubées à 4°C après introduction de [$^3$H]flunitrazepam, seul ou avec des quantités croissantes du composé à tester. On détermine par scintillation liquide, la quantité de produit marqué fixé sur les membranes après leur séparation du milieu d'incubation par fixation sur filtre de fibres de verre et on calcule par la méthode logit-log la CI50; la fixation non spécifique du flunitrazépam est déterminée en introduisant 2 µM de clonazépam.

Les résultats de ces essais pour des composés représentatifs de l'invention figurent dans le tableau V.

TABLEAU V

| SR | CI 50 (nM) (périphérique) | CI 50 (nM) (central) |
|---|---|---|
| 26241 | 3 | >20000 |
| 26399 | 7 | 12700 |
| 26310 | 1 | >20000 |
| 26351 | 6 | 30000 |
| 26377 | 1 | >20000 |
| 26421 | 5 | 53000 |
| 26492 | 1 | >20000 |
| 26522 | 11 | >20000 |
| 26552 | 31 | 12400 |
| 26487 | 39 | >20000 |
| 26378 | 1 | >20000 |
| 26579 | 1 | >20000 |
| 26493 | 12 | >20000 |
| 26449 | 1 | >20000 |
| 26362 | 3 | >20000 |
| 26423 | 1 | 12600 |
| 26306 | 11 | >20000 |
| 26319 | 8 | 14000 |
| 26269 | 16 | >20000 |
| 26641 | 356 | >20000 |
| 26920 | 18 | >20000 |
| 26619 | 132 | >20000 |
| 26632 | 101 | >20000 |
| 26869 | 172 | >20000 |
| 26555 | 5 | >20000 |
| 26275 | 22 | >20000 |
| 26529 | 16 | >20000 |
| 26581 | 43 | >10000 |
| 26620 | 2,8 | >10000 |

TABLEAU V (suite I)

| SR | CI 50 (nM) (périphérique) | CI 50 (nM) (central) |
|---|---|---|
| 26676 | 1,1 | >10000 |
| 26706 | 4 | >10000 |
| 26778 | 27 | >10000 |
| 26794 | 11 | >10000 |
| 26841 | 17 | >10000 |
| 26846 | 2,9 | >10000 |
| 26876 | 0,5 | >10000 |
| 27195 | 1 | >10000 |
| 27196 | 0,8 | >10000 |
| 27271 | 68 | >10000 |
| 26199 | 13 | 1640 |
| 26290 | 10 | >10000 |
| 26307 | 175 | >10000 |
| 26286 | 6 | 5480 |
| 26494 | 2 | 13000 |
| 26485 | 40 | >10000 |
| 26386 | 16 | >10000 |
| 26332 | 29 | >10000 |
| 26361 | 16 | >10000 |
| 26276 | 0,3 | 13400 |
| 26336 | 0,3 | 2120 |
| 26409 | 84 | >10000 |
| 26554 | 28 | >10000 |
| 26412 | 0,4 | >10000 |
| 26397 | 4 | >10000 |
| 26226 | 4 | 11800 |
| 26450 | 2 | >10000 |
| 26455 | 1 | >10000 |
| 26517 | 27 | >10000 |

TABLEAU V (suite 2)

| SR | CI 50 (nM) (PERIPHERIQUE) | CI 50 (nM) (CENTRAL) |
|---|---|---|
| 26360 | 89 | >10000 |
| 26304 | 13 | 14500 |
| 26439 | 89 | >10000 |
| 26588 | 2,1 | >10000 |
| 26610 | 35 | >10000 |
| 26643 | 1,2 | >10000 |
| 26858 | 0,77 | >10000 |
| 26919 | 52 | >10000 |
| 26554 | 28 | >10000 |
| 26830 | 0,13 | >10000 |
| 26698 | 1,95 | >10000 |
| 26878 | 246 | >10000 |
| 26640 | 46 | 3550 |
| 26651 | 0,93 | 8490 |
| 26847 | 1,59 | >10000 |
| 27161 | 2,3 | >10000 |
| 26980 | 1,31 | 9750 |
| 26803 | 7,1 | 5430 |
| 26731 | 2,74 | >10000 |
| 26710 | 3,2 | >10000 |
| 26578 | 330 | 48900 |
| 27181 | 0,89 | >10000 |
| 27153 | 1,62 | 47900 |
| 26918 | 0,16 | 1680 |
| 26708 | 2,8 | >10000 |
| 26947 | 0,79 | 18600 |
| 26838 | 10,8 | 7650 |
| PK11195 | 1,3 | 21400 |
| RO5-4864 | 44 | inactif |
| DIAZEPAM | 523 | 11 |
| CHLORDIAZE-POXIDE | inactif | 654 |

On a aussi étudié l'affinité in vivo pour les récepteurs de type périphérique de certains composés de l'invention. Les essais ont été effectués chez la souris, en utilisant le ligand [$^3$H] PK11195.

Les produits à tester, en suspension dans une solution aqueuse de carboxyméthylcellulose (1% p/v) sont administrés, par voie orale à des groupes de 4 souris, 25 mn avant l'injection intra-veineuse de [$^3$H] PK11195, à raison de 200 µCi/kg (activité spécifique 66 Ci/mmole). 5 mn après l'injection, les animaux sont sacrifiés par décapitation et les différents organes sont prélevés et broyés dans 10 ml de tampon à base de

Tris-HCl (50mM; pH = 7,4) pour donner des homogénats.

Les groupes témoins reçoivent uniquement la solution de carboxyméthylcellulose et l'injection de [$^3$H]-PK11195.

On détermine pour chaque organe par scintigraphie :

1 - la quantité de ligand radioactif présente dans un organe en mesurant la radioactivité d'une partie aliquote de l'homogénat.

2 - la quantité de ligand radioactif fixée sur les tissus membranaires d'un organe en mesurant la radioactivité fixée sur un filtre de fibres de verre par filtration d'une autre partie aliquote de l'homogénat.

3 - la quantité de ligand radioactif fixée non spécifiquement sur les tissus membranaires en incubant un homogénat témoin avec un excès de RO54864, ligand connu, suivi de la fixation des membranes sur filtre et de la mesure de la radioactivité restant sur les membranes.

L'inhibition de la fixation spécifique, notée FS, est égale à la différence des radioactivités mesurées en 2 et 3 divisée par la mesure en 1.

Les pourcentages d'inhibition de la fixation dans différents organes déterminés pour des composés de l'invention et les produits de référence figurent dans le tableau VI; ils sont donnés par la formule :

$$100 \times \frac{\text{FS (témoin)} - \text{FS (produit)}}{\text{FS (témoin)}}$$

TABLEAU VI

| SR | Dose (mg/kg) | % inhibition | | | | |
|---|---|---|---|---|---|---|
| | | cerveau | rate | thymus | rein | coeur |
| 26276 | 8 | 78 | 37 | 34 | 69 | 30 |
| 26830 | 25 | 64 | 26 | nd | nd | nd |
| 26412 | 25 | 79 | 62 | nd | nd | nd |
| 26876 | 25 | 84 | 64 | nd | nd | nd |
| 26858 | 25 | 95 | 84 | nd | nd | nd |
| 27161 | 25 | 92 | 78 | nd | nd | nd |
| 26846 | 25 | 91 | 80 | nd | nd | nd |
| 27153 | 25 | 87 | 52 | nd | nd | nd |
| 26310 | 25 | 76 | 52 | 31 | 32 | 39 |
| PK 11195 | 15 | 71 | 29 | 30 | 54 | 27 |
| RO5-4864 | 15 | 74 | 11 | 28 | 73 | 27 |
| nd = non déterminé | | | | | | |

**Revendications**

**Revendications pour les Etats contractants suivants : AT, BE, CH, DE, FR, GB, IT, LI, LU, NL, SE**

**1.** Composés de formule I

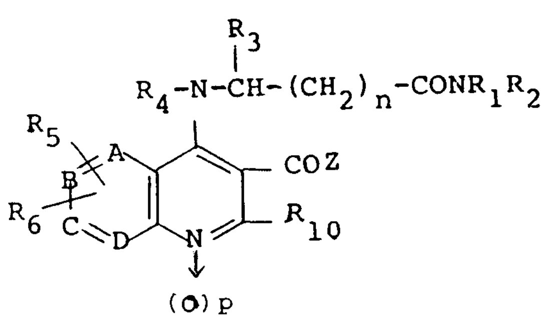

I

dans laquelle $R_1$ et $R_2$ identiques ou différents, représentent chacun l'atome d'hydrogène, un groupe alkyle en $C_1$ à $C_6$ ou alkényle en $C_2$ à $C_6$, un groupe phényle ou benzyle, ou $R_1$ et $R_2$ forment avec l'atome d'azote auquel ils sont rattachés un hétérocycle saturé choisi parmi pipéridine, pyrrolidine, morpholine et pipérazine,
$R_3$ représente l'atome d'hydrogène, un groupe alkyle en $C_1$ à $C_6$, phénylalkyle en $C_7$ à $C_9$, ou phényle
$R_4$ représente l'atome d'hydrogène ou un groupe alkyle en $C_1$ à $C_4$,
$R_5$ et $R_6$, identiques ou différents représentent chacun un atome d'hydrogène ou d'halogène, un groupe alkyle ou alkoxy en $C_1$ à $C_3$, un groupe nitro, trifluorométhyle,
ou ensemble forment un groupe méthylènedioxy,
Z représente $OR_7$ dans lequel $R_7$ représente l'atome d'hydrogène ou un groupe alkyle en $C_1$ à $C_6$; $NR_8R_9$ dans lequel $R_8$ et $R_9$ représentent chacun l'atome d'hydrogène, un groupe alkyle en $C_1$ à $C_4$, un groupe phényle ou benzyle; un groupe alkyle en $C_1$ à $C_4$; un groupe benzyle; un groupe aryle choisi parmi les groupes phényle, pyridyle, pyrrolyle, furyle, thiényle et imidazolyle,
$R_{10}$ représente l'atome d'hydrogène, un groupe alkyle en $C_1$ à $C_4$ ou un groupe phényle,
étant entendu que lorsque Z n'est pas un groupe benzyle ou aryle, $R_3$ ne représente pas H,
les groupes phényle et benzyle pouvant être substitués par les atomes d'halogène, les groupes alkoxy, alkyles et thioalkyles en $C_1$ à $C_3$, les groupes nitro, trifluorométhyle et hydroxy, les groupes alkyle et alkoxy pouvant être linéaires, ramifiés ou cycliques
n représente 0,1 ou 2, p représente 0 ou 1 et
l'un des symboles A,B,C,D représente N et les autres CH ou A,B,C,D représentent chacun CH,
sous forme d'un racémique ou des énantiomères,
ainsi que leurs sels d addition avec les acides ou les bases pharmaceutiquement acceptables.

2. Composés selon la revendication 1 répondant à la formule I dans laquelle Z est un groupe phényle substitué ou non.

3. Composés selon la revendication 2, dans laquelle Z est un groupe phényle non substitué.

4. Composés selon la revendication 1, répondant à la formule I dans laquelle Z est un groupe pyridyle, pyrrolyle, furyle, thiényle ou imidazolyle.

5. Composés selon la revendication 1, répondant à la formule I dans laquelle Z est un groupe $OR_7$.

6. Composés selon la revendication 5, dans laquelle $R_7$ représente un groupe alkyle.

7. Composés selon la revendication 5, dans laquelle $R_7$ représente un groupe alkyle en $C_1$ à $C_3$.

8. Composés selon l'une des revendications 1 à 7 de formule I dans laquelle A, B, C et D représentent chacun CH.

9. Composés selon l'une des revendications 1 à 7 de formule I dans laquelle l'un des symboles A, B, C ou D représente N.

10. Composés selon la revendication 9 dans laquelle A représente N.

11. Composés selon la revendication 1 de formule I dans laquelle $R_1$ et $R_2$ représentent chacun un groupe alkyle en $C_1$ à $C_6$, $R_3$ représente un groupe alkyle en $C_1$ à $C_3$, $R_{10}$ représente l'hydrogène et Z représente $OR_7$ avec $R_7$ représentant un groupe alkyle en $C_1$ à $C_6$.

12. Composés selon la revendication 11 dans lesquels $R_4$ représente l'hydrogène.

13. Composés selon la revendication 11 dans lesquels $R_4$ représente l'hydrogène et le symbole C est substitué.

14. Composés selon la revendication 1 de formule I dans laquelle $R_1$ représente un groupe alkyle, $R_2$ et Z représentent chacun un groupe phényle pouvant être substitué.

**15.** Composés selon la revendication 1 de formule I dans laquelle $R_1$ représente un groupe alkyle $R_2$ et Z représentent un groupe phényle pouvant être substitué, $R_3$ et $R_{10}$ représentent chacun l'atome d'hydrogène.

**16.** Composés selon l'une des revendications 14 et 15 pour lesquels les symboles A, B, C et D représentent chacun CH.

**17.** Composés selon l'une des revendications 14 et 15 pour lesquels le symbole A représente N et B, C et D représentent CH.

**18.** Composé selon la revendication 1, ledit composé étant le [chloro-7(N,N-dipropylcarbamoyl-1 éthylamino)-4 quinoléinyl-3] carboxylate d'éthyle et ses sels d'addition avec les acides pharmacologiquement acceptables.

**19.** Composé selon la revendication 1, ledit composé étant le N-méthyl N-(chloro-4)phényl [benzoyl-3 chloro-7 quinoléinyl-4] aminoacétamide et ses sels d'addition avec les acides pharmacologiquement acceptables.

**20.** Composé selon la revendication 1, ledit composé étant le N-méthyl N-(chloro-4)phényl (benzoyl-3 naphtyridine-1,5)yl-4 aminoacétamide et ses sels d'addition avec les acides pharmacologiquement acceptables.

**21.** Procédé de préparation des composés de formule I selon la revendication 1, caractérisé en ce que l'on fait réagir l'amine de formule II :

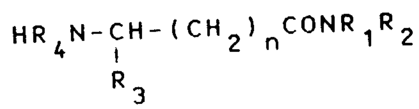

avec un composé de formule III :

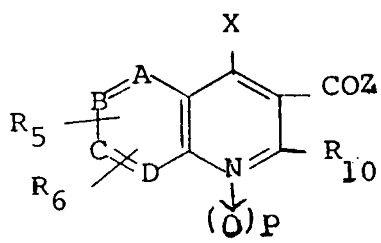

III

dans lequelles $R_1$, $R_2$, $R_3$, $R_4$, $R_5$, $R_6$, $R_{10}$, Z, A, B, C, D, n et p ont les mêmes significations que dans la revendication 1 et X représente un atome d'halogène ou un groupe sulfonate.

**22.** Composition pharmaceutique comprenant comme principe actif un composé selon l'une quelconque des revendications 1 à 20 ainsi qu'un excipient compatible.

**Revendications pour les Etats contractants suivants : ES, GR**

**1.** Procédé de préparation de composés de formule I

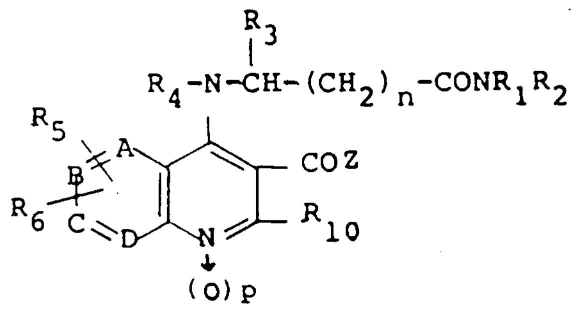

I

dans laquelle $R_1$ et $R_2$ identiques ou différents, représentent chacun l'atome d'hydrogène, un groupe alkyle en $C_1$ à $C_6$ ou alkényle en $C_2$ à $C_6$, un groupe phényle ou benzyle, ou $R_1$ et $R_2$ forment avec l'atome d'azote auquel ils sont rattachés un hétérocycle saturé choisi parmi pipéridine, pyrrolidine, morpholine et pipérazine,
$R_3$ représente l'atome d'hydrogène, un groupe alkyle en $C_1$ à $C_6$, phénylalkyle en $C_7$ à $C_9$, ou phényle
$R_4$ représente l'atome d hydrogène ou un groupe alkyle en $C_1$ à $C_4$,
$R_5$ et $R_6$, identiques ou différents représentent chacun un atome d'hydrogène ou d'halogène, un groupe alkyle ou alkoxy en $C_1$ à $C_3$, un groupe nitro, trifluorométhyle,
ou ensemble forment un groupe méthylènedioxy,
Z représente $OR_7$ dans lequel $R_7$ représente l'atome d hydrogène ou un groupe alkyle en $C_1$ à $C_6$; $NR_8R_9$ dans lequel $R_8$ et $R_9$ représentent chacun l'atome d'hydrogène, un groupe alkyle en $C_1$ à $C_4$, un groupe phényle ou benzyle; un groupe alkyle en $C_1$ à $C_4$; un groupe benzyle ; un groupe aryle choisi parmi les groupes phényle, pyridyle, pyrrolyle, furyle, thiényle et imidazolyle,
$R_{10}$ représente l'atome d'hydrogène, un groupe alkyle en $C_1$ à $C_4$ ou un groupe phényle,
étant entendu que lorsque Z n'est pas un groupe benzyle ou aryle, $R_3$ ne représente pas H,
les groupes phényle et benzyle pouvant être substitués par les atomes d'halogène, les groupes alkoxy, alkyles et thioalkyles en $C_1$ à $C_3$, les groupes nitro trifluorométhyle et hydroxy, les groupes alkyle et alkoxy pouvant être linéaires, ramifiés ou cycliques
n représente 0,1 ou 2, p représente 0 ou 1 et
l'un des symboles A,B,C,D représente N et les autres CH ou A,B,C,D représentent chacun CH,
sous forme d'un racémique ou des énantiomères,
ainsi que leurs sels d'addition avec les acides ou les bases pharmaceutiquement acceptables, caractérisé en ce que l'on fait réagir l'amine de formule II :

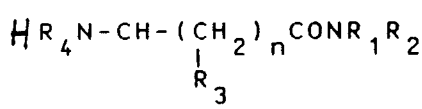

avec un composé de formule III :

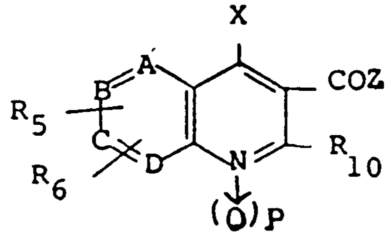

III

dans lequelles $R_1$, $R_2$, $R_3$, $R_4$, $R_5$, $R_6$, $R_{10}$, Z, A, B, C, D, n et p ont les mêmes significations que dans la formule I et X représente un atome d'halogène ou un groupe sulfonate.

**2.** Procédé selon la revendication 1, caractérisé en ce que l'on prépare les composés répondant à la formule I dans laquelle Z est un groupe phényle substitué ou non.

**3.** Pocédé selon la revendication 2, caractérisé en ce que l'on prépare les composés dans laquelle Z est un groupe phényle non substitué.

**4.** Procédé selon la revendication 1, caractérisé en ce que l'on prépare les composés répondant à la formule I dans laquelle Z est un groupe pyridyle, pyrrolyle, furyle, thiényle ou imidazolyle.

**5.** Procédé selon la revendication 1, caractérisé en ce que l'on prépare les composés répondant à la formule I dans laquelle Z est un groupe $OR_7$.

**6.** Procédé selon la revendication 5, caractérisé en ce que l'on prépare les composés de formule I dans laquelle $R_7$ représente un groupe alkyle.

**7.** Procédé selon la revendication 5, caractérisé en ce que l'on prépare les composés de formule I dans laquelle $R_7$ représente un groupe alkyle en $C_1$ à $C_3$.

**8.** Procédé selon l'une des revendications 1 à 7, caractérisé en ce que l'on prépare les composés de formule I dans laquelle A, B, C et D représentent chacun CH.

**9.** Procédé selon l'une des revendications 1 à 7 de formule I dans laquelle l'un des symboles A, B, C ou D représente N.

**10.** Procédé selon la revendication 9, caractérisé en ce que l'on prépare les composés de formule I dans laquelle A représente N.

**11.** Procédé selon la revendication 1, caractérisé en ce que l'on prépare les composés de formule I dans laquelle $R_1$ et $R_2$ représentent chacun un groupe alkyle en $C_1$ à $C_6$, $R_3$ représente un groupe alkyle en $C_1$ à $C_3$, $R_{10}$ représente l'hydrogène et Z représente $OR_7$ avec $R_7$ représentant un groupe alkyle en $C_1$ à $C_6$.

**12.** Procédé selon la revendication 11, caractérisé en ce que l'on prépare les composés dans lesquels $R_4$ représente l'hydrogène.

**13.** Procédé selon la revendication 11 dans lesquels $R_4$ représente l'hydrogène et le symbole C est substitué.

**14.** Procédé selon la revendication 1, caractérisé en ce que l'on prépare les composés de formule I dans laquelle $R_1$ représente un groupe alkyle $R_2$ et Z représentent chacun un groupe phényle pouvant être substitué.

**15.** Procédé selon la revendication 1, caractérisé en ce que l'on prépare les composés de formule I dans laquelle $R_1$ représente un groupe alkyle $R_2$ et Z représentent un groupe phényle pouvant être substitué, $R_3$ et $R_{10}$ représentent chacun l'atome d'hydrogène.

**16.** Procédé selon l'une des revendications 14 et 15, caractérisé en ce que l'on prépare les composés pour lesquels les symboles A, B, C et D représentent chacun CH.

**17.** Procédé selon l'une des revendications 14 et 15 pour lesquels le symbole A représente N et B, C et D représentent CH.

**18.** Procédé selon la revendication 1, caractérisé en ce que l'on prépare le [chloro-7(N,N-dipropylcarbamoyl-1 éthylamino)-4 quinoléinyl-3] carboxylate d'éthyle et ses sels d'addition avec les acides pharmacologiquement acceptables.

**19.** Procédé selon la revendication 1, caractérisé en ce que l'on prépare N-méthyl N-(chloro-4)phényl [benzoyl-3 chloro-7 quinoléinyl-4] aminoacétamide et ses sels d addition avec les acides pharmacologiquement acceptables.

**20.** Procédé selon la revendication 1, caractérisé en ce que l'on prépare N-méthyl N-(chloro-4)phényl (benzoyl-3 naphtyridine-1,5)yl-4 aminoacétamide et ses sels d'addition avec les acides pharmacologiquement acceptables.

**Claims**

**Claims for the following Contracting States : AT, BE, CH, DE, FR, GB, IT, LI, LU, NL, SE**

**1.** Compounds of formula I

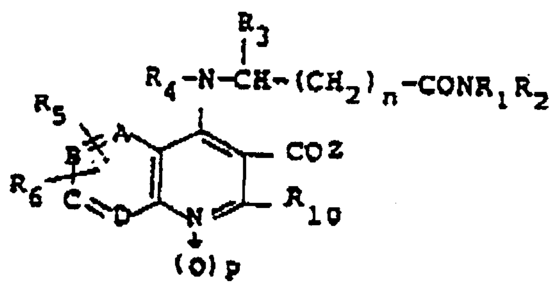

I

in which $R_1$ and $R_2$, which my be identical or different, each represent a hydrogen atom, a $C_1$ to $C_6$ alkyl or $C_2$ to $C_6$ alkenyl group, a phenyl or benzyl group, or $R_1$ and $R_2$, together with the nitrogen atom to which they are attached, form a saturated heterocycle chosen from piperidine, pyrrolidine, morpholine and piperazine,
$R_3$ represents a hydrogen atom, a $C_1$ to $C_6$ alkyl, a $C_7$ to $C_9$ phenylalkyl or a phenyl group,
$R_4$ represents a hydrogen atom or a $C_1$ to $C_4$ alkyl group,
$R_5$ and $R_6$, which may be identical or different, each represent a hydrogen or halogen atom, a $C_1$ to $C_3$ alkyl or alkoxy group, a nitro or trifluoromethyl group,
or together form a methylenedioxy group,
Z represents $OR_7$, in which $R_7$ represents a hydrogen atom or a $C_1$ to $C_6$ alkyl group; $NR_8R_9$ in which $R_8$ and $R_9$ each represent a hydrogen atom, a $C_1$ to $C_4$ alkyl group or a phenyl or benzyl group; a $C_1$ to $C_4$ alkyl group; a benzyl group; an aryl group chosen from phenyl, pyridyl, pyrrolyl, furyl, thienyl and imidazolyl groups,
$R_{10}$ represents a hydrogen atom, a $C_1$ to $C_4$ alkyl group or a phenyl group,
it being understood that when Z is not a benzyl or aryl group $R_3$ does not represent H,
it being possible for the phenyl and benzyl groups to be substituted with halogen atoms, $C_1$ to $C_3$ alkoxy, alkyl and thioalkyl groups, nitro, trifluoromethyl and hydroxyl groups or alkyl and alkoxy groups which may be linear, branched or cyclic,
n represents 0, 1 or 2, p represents 0 or 1 and one of the symbols A, B, C and D represents N and the others CH, or A, B, C and D each represent CH,
in the form of a racemate or of enantiomers,
as well as their addition salts with pharmaceutically acceptable acids or bases.

**2.** Compounds according to Claim 1, corresponding to the formula I, in which Z is a substituted or unsubstituted phenyl group.

**3.** Compounds according to Claim 2, in which Z is an unsubstituted phenyl group.

**4.** Compounds according to Claim 1, corresponding to the formula I, in which Z is a pyridyl, pyrrolyl, furyl, thienyl or imidazolyl group.

**5.** Compounds according to Claim 1, corresponding to the formula I, in which Z in a group $OR_7$.

**6.** Compounds according to Claim 5, in which $R_7$ represents an alkyl group.

**7.** Compounds according to Claim 5, in which $R_7$ represents a $C_1$ to $C_3$ alkyl group.

**8.** Compounds according to one of Claims 1 to 7, of formula I, in which A, B, C and D each represent CH.

**9.** Compounds according to one of Claims 1 to 7, of formula I, in which one of the symbols A, B, C or D represents N.

**10.** Compounds according to Claim 9, in which A represents N are prepared.

**11.** Compounds according to Claim 1, of formula I, in which $R_1$ and $R_2$ each represent a $C_1$ to $C_6$ alkyl group, $R_3$ represents a $C_1$ to $C_3$ alkyl group, $R_{10}$ represents hydrogen and Z represents $OR_7$ with $R_7$ representing a $C_1$ to $C_6$ alkyl group.

**12.** Compounds according to Claim 11, in which $R_4$ represents hydrogen.

**13.** Compounds according to Claim 11, in which $R_4$ represents hydrogen and the symbol C is substituted.

**14.** Compounds according to Claim 1, in which $R_1$ represents an alkyl group and $R_2$ and Z each represent a phenyl group which may be substituted.

**15.** Compounds according to Claim 1, of formula I, in which $R_1$ represents an alkyl group, $R_2$ and Z represent a phenyl group which may be substituted and $R_3$ and $R_{10}$ each represent a hydrogen atom.

**16.** Compounds according to either of Claims 14 and 15, for which the symbols A, B, C and D each represent CH.

**17.** Compounds according to either of Claims 14 and 15, characterized in that the compounds for which the symbol A represents N and B, C and D represent CH.

**18.** Compound according to Claim 1, the said compound being ethyl 7-chloro-4-(N,N-dipropylcarbamoyl-1-ethylamino)-3-quinolinecarboxylate, and its addition salts with pharmacologically acceptable acids.

**19.** Compound according to Claim 1, the said compound being N-methyl-N-(4-chlorophenyl)-(3-benzoyl-7-chloro-4-quinolyl)aminoacetamide, and its addition salts with pharmacologically acceptable acids.

**20.** Compound according to Claim 1, the said compound being N-methyl-N-(4-chlorophenyl)-(3-benzoyl-1,5-naphthyridin-4-yl)aminoacetamide, and its addition salts with pharmacologically acceptable acids.

**21.** Process for the preparation of the compounds of formula I according to Claim 1, characterized in that the amine of formula II:

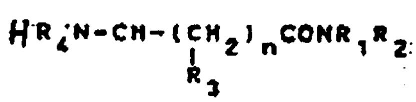

is reacted with a compound of formula III:

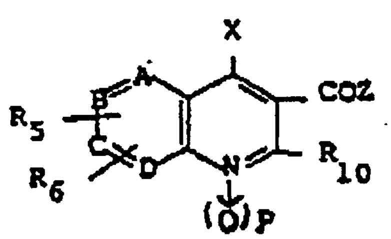

III

in which formulae $R_1$, $R_2$, $R_3$, $R_4$, $R_5$, $R_6$, $R_{10}$, Z, A, B, C, D, n and p have the same meanings is in Claim I and X represents a halogen atom or a sulphonate group.

**22.** Pharmaceutical composition comprising as active principle a compound according to any one of Claims 1 to 20, together with a compatible excipient.

**Claims for the following Contracting States : ES, GR**

**1.** Process for the preparation of compounds of formula I

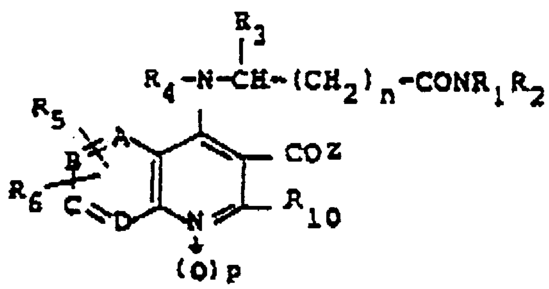

I

in which $R_1$ and $R_2$, which may be identical or different, each represent a hydrogen atom, a $C_1$ to $C_6$ alkyl or $C_2$ to $C_6$ alkenyl group, a phenyl or benzyl group, or $R_1$ and $R_2$, together with the nitrogen atom to which they are attached, form a saturated heterocycle chosen from piperidine, pyrrolidine, morpholine and piperazine,
$R_3$ represents a hydrogen atom, a $C_1$ to $C_6$ alkyl, a $C_7$ to $C_9$ phenylalkyl or a phenyl group,
$R_4$ represents a hydrogen atom or a $C_1$ to $C_4$ alkyl group,
$R_5$ and $R_6$, which may be identical or different, each represent a hydrogen or halogen atom, a $C_1$ to $C_3$ alkyl or alkoxy group, a nitro or trifluoromethyl group,
or together form a methylenedioxy group,
Z represents $OR_7$, in which $R_7$ represents a hydrogen atom or a $C_1$ to $C_6$ alkyl group; $NR_8R_9$ in which $R_8$ and $R_9$ each represent a hydrogen atom, a $C_1$ to $C_4$ alkyl group or a phenyl or benzyl group; a $C_1$ to $C_4$ alkyl group; a benzyl group; an aryl group chosen from phenyl, pyridyl, pyrrolyl, furyl, thienyl and imidazolyl groups,
$R_{10}$ represents a hydrogen atom, a $C_1$ to $C_4$ alkyl group or a phenyl group,
it being understood that when Z is not a benzyl or aryl group $R_3$ does not represent H,
it being possible for the phenyl and benzyl groups to be substituted with halogen atoms, $C_1$ to $C_3$ alkoxy, alkyl and thioalkyl groups, nitro, trifluoromethyl and hydroxyl groups or alkyl and alkoxy groups which may be linear, branched or cyclic,
n represents 0, 1 or 2, p represents 0 or 1 and one of the symbols A, B, C and D represents N and the others CH, or A, B, C and D each represent CH,
in the form of a racemate or of enantiomers,
as well as their addition salts with pharmaceutically acceptable acids or bases, characterized in that the amine of formula II:

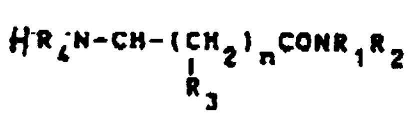

is reacted with a compound of formula III:

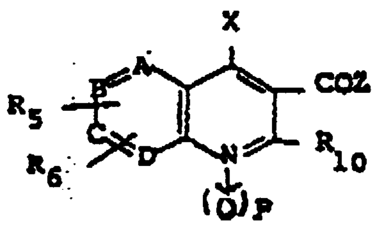

III

in which formulae $R_1$, $R_2$, $R_3$, $R_4$, $R_5$, $R_6$, $R_{10}$, Z, A, B, C, D, n and p have the same meanings as in formula I and X represents a halogen atom or a sulphonate group.

**2.** Process according to Claim 1, characterized in that the compounds corresponding to the formula I in which Z is a substituted or unsubstituted phenyl group are prepared.

**3.** Process according to Claim 2, characterized in that the compounds in which Z is an unsubstituted phenyl group are prepared.

**4.** Process according to Claim 1, characterized in that the compounds corresponding to the formula I in which Z is a pyridyl, pyrrolyl, furyl, thienyl or imidazolyl group are prepared.

**5.** Process according to Claim 1, characterized in that the compounds corresponding to the formula I in which Z is a group $OR_7$ are prepared.

**6.** Process according to Claim 5, characterized in that the compounds of formula I in which $R_7$ represents an alkyl group are prepared.

**7.** Process according to Claim 5, characterized in that the compounds of formula I in which $R_7$ represents a $C_1$ to $C_3$ alkyl group are prepared.

**8.** Process according to one of Claims 1 to 7, characterized in that the compounds of formula I in which A, B, C and D each represent CH are prepared.

**9.** Process according to one of Claims 1 to 7, characterized in that the compounds of formula I in which one of the symbols A, B, C or D represents N are prepared.

**10.** Process according to Claim 9, characterized in that the compounds of formula I in which A represents N are prepared.

**11.** Process according to Claim 1, characterized in that the compounds of formula I in which $R_1$ and $R_2$ each represent a $C_1$ to $C_6$ alkyl group, $R_3$ represents a $C_1$ to $C_3$ alkyl group, $R_{10}$ represents hydrogen and Z represents $OR_7$ with $R_7$ representing a $C_1$ to $C_6$ alkyl group are prepared.

**12.** Process according to Claim 11, characterized in that the compounds in which $R_4$ represents hydrogen are prepared.

**13.** Process according to Claim 11, in which $R_4$ represents hydrogen and the symbol C is substituted.

**14.** Process according to Claim 1, characterized in that the compounds of formula I in which $R_1$ represents an alkyl group and $R_2$ and Z each represent a phenyl group which may be substituted are prepared.

**15.** Process according to Claim 1, characterized in that the compounds of formula I in which $R_1$ represents an alkyl group, $R_2$ and Z represent a phenyl group which may be substituted and $R_3$ and $R_{10}$ each represent a hydrogen atom are prepared.

**16.** Process according to either of Claims 14 and 15, characterized in that the compounds for which the symbols A, B, C and D each represent CH are prepared.

**17.** Process according to either of Claims 14 and 15, characterized in that the compounds for which the symbol A represents N and B, C and D represent CH are prepared.

**18.** Process according to Claim 1, characterized in that ethyl 7-chloro-4-(N,N-dipropylcarbamoyl-1-ethylamino)-3-quinolinecarboxylate and its addition salts with pharmacologically acceptable acids are prepared.

**19.** Process according to Claim 1, characterized in that N-methyl-N-(4-chlorophenyl)-(3-benzoyl-7-chloro-4-quinolyl)aminoacetamide and its addition salts with pharmacologically acceptable acids are prepared.

**20.** Process according to Claim 1, characterized in that N-methyl-N-(4-chlorophenyl)-(3-benzoyl-1,5-naphthyridin-4-yl)aminoacetamide and its addition salts with pharmacologically acceptable acids are prepared.

**Patentansprüche**
**Patentansprüche für folgende Vertragsstaaten : AT, BE, CH, DE, FR, GB, IT, LI, LU, NL, SE**

**1.** Verbindungen der Formel I

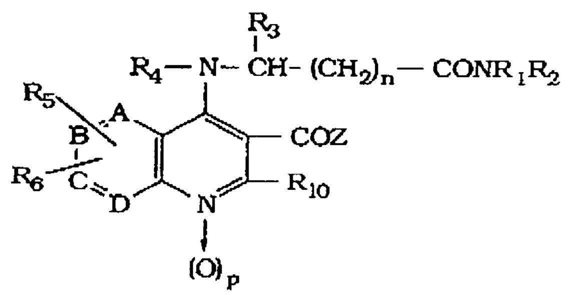

I

in der $R_1$ und $R_2$, die gleichartig oder verschieden sein können, jeweils ein Wasserstoffatom, eine $C_1$-$C_6$-Alkylgruppe, eine $C_2$-$C_6$-Alkenylgruppe, eine Phenylgruppe oder eine Benzylgruppe oder $R_1$ und $R_2$ gemeinsam mit dem Stickstoffatom, an das sie gebunden sind, einen aus Piperidin, Pyrrolidin Morpholin und Piperazin ausgewählten gesättigten Heterocyclus.
$R_3$ ein Wasserstoffatom, eine $C_1$-$C_6$-Alkylgruppe, eine $C_7$-$C_9$-Phenylalkylgruppe oder eine Phenylgruppe,
$R_4$ ein Wasserstoffatom oder eine $C_1$-$C_4$-Alkylgruppe,
$R_5$ und $R_6$, die gleichartig oder verschieden sein können, jeweils ein Wasserstoffatom, ein Halogenatom, eine $C_1$-$C_3$-Alkylgruppe, eine $C_1$-$C_3$-Alkoxygruppe, eine Nitrogruppe, eine Trifluormethylgruppe oder gemeinsam eine Methylendioxygruppe.
Z $OR_7$, worin $R_7$ ein Wasserstoffatom oder eine $C_1$-$C_6$-Alkylgruppe; $NR_8R_9$, worin $R_8$ und $R_9$ jeweils ein Wasserstoffatom, eine $C_1$-$C_4$-Alkylgruppe, eine Phenylgruppe oder eine Benzylgruppe; eine $C_1$-$C_4$-Alkylgruppe; eine Benzylgruppe; oder eine aus Phenylgruppen, Pyridylgruppen, Pyrrolylgruppen, Furylgruppen, Thienylgruppen und Imidazolylgruppen ausgewählte Arylgruppe,
$R_{10}$ ein Wasserstoffatom, eine $C_1$-$C_4$-Alkylgruppe oder eine Phenylgruppe, mit der Maßgabe, daß, wenn Z keine Benzyl- oder Arylgruppe darstellt. $R_3$ kein Wasserstoffatom bedeutet,
die Phenyl- und Benzylgruppen durch Halogenatome, Alkoxy-, Alkyl- und Thioalkyl-gruppen mit 1 bis 3 Kohlenstoffatomen, Nitrogruppen, Trifluormethylgruppen und Hydroxylgruppen substituiert sein können und die Alkyl- und Alkoxygruppen geradkettig, verzweigt oder cyclisch sein können,
n 0, 1 oder 2, p 0 oder 1 und
eines der Symbole A, B, C, D N und die anderen CH oder A, B, C, D jeweils CH bedeuten,
in Form der Racemate oder der Enantiomeren, sowie ihre Additionssalze mit pharmazeutisch annehmbaren Säuren oder Basen.

**2.** Verbindungen nach Anspruch 1 der Formel I, worin Z eine substituierte oder nichtsubstituierte Phenylgruppe bedeutet.

**3.** Verbindungen nach Anspruch 2, worin Z eine nichtsubstituierte Phenylgruppe bedeutet.

4. Verbindungen nach Anspruch 1 der Formel I, worin Z eine Pyridyl-, Pyrrolyl-, Furyl-, Thienyl- oder Imidazolylgruppe bedeutet.

5. Verbindungen nach Anspruch 1 der Formel I, worin Z eine Gruppe $OR_7$ bedeutet.

6. Verbindungen nach Anspruch 5, worin $R_7$ eine Alkylgruppe bedeutet.

7. Verbindungen nach Anspruch 5, worin $R_7$ eine $C_1$-$C_3$-Alkylgruppe bedeutet.

8. Verbindungen nach einem der Ansprüche 1 bis 7 der Formel I, worin A, B, C und D jeweils CH bedeuten.

9. Verbindungen nach einem der Ansprüche 1 bis 7 der Formel I, worin eines der Symbole A, B, C oder D N bedeutet.

10. Verbindungen nach Anspruch 9, worin A N bedeutet.

11. Verbindungen nach Anspruch 1 der Formel I, worin $R_1$ und $R_2$ jeweils eine $C_1$-$C_6$-Alkylgruppe, $R_3$ eine $C_1$-$C_3$-Alkylgruppe, $R_{10}$ Wasserstoff und Z $OR_7$ bedeuten, worin $R_7$ eine $C_1$-$C_6$-Alkylgruppe darstellt.

12. Verbindungen nach Anspruch 11, worin $R_4$ Wasserstoff bedeutet.

13. Verbindungen nach Anspruch 11, worin $R_4$ Wasserstoff bedeutet und das Symbol C substituiert ist.

14. Verbindungen nach Anspruch 1 der Formel I, worin $R_1$ eine Alkylgruppe und $R_2$ und Z jeweils eine gegebenenfalls substituierte Phenylgruppe bedeuten.

15. Verbindungen nach Anspruch 1 der Formel I, worin $R_1$ eine Alkylgruppe, $R_2$ und Z eine gegebenenfalls substituierte Phenylgruppe und $R_3$ und $R_{10}$ jeweils ein Wasserstoffatom bedeuten.

16. Verbindungen nach einem der Ansprüche 14 und 15, worin die Symbole A, B, C und D jeweils CH bedeuten.

17. Verbindungen nach einem der Ansprüche 14 und 15, worin das Symbol A N und B, C und D CH bedeuten.

18. Verbindung nach Anspruch 1, nämlich [7-Chlor-4-(1-N,N-dipropylcarbamoyl-ethylamino)-chinolin-3-yl]-carbonsäureethylester und dessen Additionssalze mit pharmazeutisch annehmbaren Säuren.

19. Verbindung nach Anspruch 1, nämlich N-Methyl-N-(4-chlor)-phenyl-[3-benzoyl-7-chlor-chinolin-4-yl]-aminoacetamid und dessen Additionssalze mit pharmazeutisch annehmbaren Säuren.

20. Verbindung nach Anspruch 1, nämlich N-Methyl-N-(4-chlor)-phenyl-(3-benzoyl-1,5-naphthyridin)-4-yl-aminoacetamid und dessen Additionssalze mit pharmazeutisch annehmbaren Säuren.

21. Verfahren zur Herstellung der Verbindungen der Formel I nach Anspruch 1, **dadurch gekennzeichnet,** daß man das Amin der Formel II:

$$HR_4N-\underset{\displaystyle R_3}{\underset{|}{CH}}-(CH_2)_nCONR_1R_2 \qquad II$$

mit einer Verbindung der Formel III:

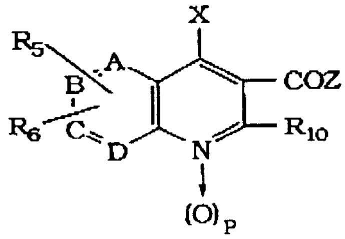

III

worin $R_1$, $R_2$, $R_3$, $R_4$, $R_5$, $R_6$, $R_{10}$, Z, A, B, C, D, n und p die in Anspruch 1 angegebenen Bedeutungen besitzen und X ein Halogenatom oder eine Sulfonatgruppe bedeutet, umsetzt.

**22.** Pharmazeutische Zubereitung enthaltend als Wirkstoff eine Verbindung nach einem der Ansprüche 1 bis 20 sowie einen verträglichen Träger.

**Patentansprüche für folgende Vertragsstaaten : ES, GR**

**1.** Verfahren zur Herstellung von Verbindungen der Formel I

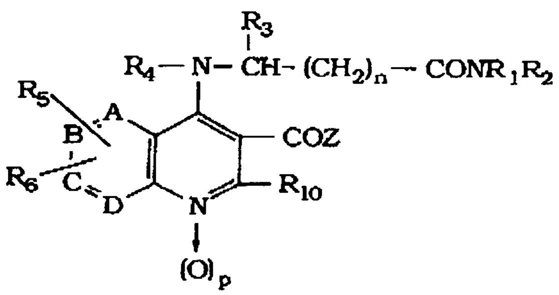

I

in der $R_1$ und $R_2$, die gleichartig oder verschieden sein können, jeweils ein Wasserstoffatom, eine $C_1$-$C_6$-Alkylgruppe, eine $C_2$-$C_6$-Alkenylgruppe, eine Phenylgruppe oder eine Benzylgruppe oder $R_1$ und $R_2$ gemeinsam mit dem Stickstoffatom, an das sie gebunden sind, einen aus Piperidin, Pyrrolidin, Morpholin und Piperazin ausgewählten gesättigten Heterocyclus,
$R_3$ ein Wasserstoffatom, eine $C_1$-$C_6$-Alkylgruppe, eine $C_7$-$C_9$-Phenylalkylgruppe oder eine Phenylgruppe,
$R_4$ ein Wasserstoffatom oder eine $C_1$-$C_4$-Alkylgruppe,
$R_5$ und $R_6$, die gleichartig oder verschieden sein können, jeweils ein Wasserstoffatom, ein Halogenatom, eine $C_1$-$C_3$-Alkylgruppe, eine $C_1$-$C_3$-Alkoxygruppe, eine Nitrogruppe, eine Trifluormethylgruppe oder gemeinsam eine Methylendioxygruppe,
Z $OR_7$, worin $R_7$ ein Wasserstoffatom oder eine $C_1$-$C_6$-Alkylgruppe; $NR_8R_9$, worin $R_8$ und $R_9$ jeweils ein Wasserstoffatom, eine $C_1$-$C_4$-Alkylgruppe, eine Phenylgruppe oder eine Benzylgruppe: eine $C_1$-$C_4$-Alkylgruppe; eine Benzylgruppe; oder eine aus Phenylgruppen, Pyridylgruppen, Pyrrolylgruppen, Furylgruppen, Thienylgruppen und Imidazolylgruppen ausgewählte Arylgruppe,
$R_{10}$ ein Wasserstoffatom, eine $C_1$-$C_4$-Alkylgruppe oder eine Phenylgruppe, mit der Maßgabe, daß, wenn Z keine Benzyl- oder Arylgruppe darstellt, $R_3$ kein Wasserstoffatom bedeutet,
die Phenyl- und Benzylgruppen durch Halogenatome, Alkoxy-, Alkyl- undThioalkyl-gruppen mit 1 bis 3 Kohlenstoffatomen, Nitrogruppen, Trifluormethylgruppen und Hydroxylgruppen substituiert sein können und die Alkyl- und Alkoxygruppen geradkettig, verzweigt oder cyclisch sein können,
n 0, 1 oder 2, p 0 oder 1 und
eines der Symbole A, B, C, D N und die anderen CH oder A, B, C, D jeweils CH bedeuten,
in Form der Racemate oder der Enantiomeren,
sowie von deren Additionssalzen mit pharmazeutisch annehmbaren Säuren oder Basen,
**dadurch gekennzeichnet,** daß man das Amin der Formel II:

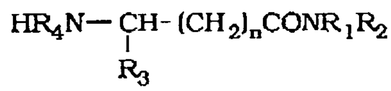

II

mit einer Verbindung der Formel III:

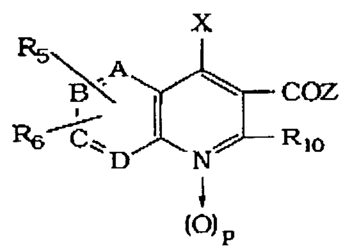

III

worin $R_1$, $R_2$, $R_3$, $R_4$, $R_5$, $R_6$, $R_{10}$, Z, A, B, C, D, n und p die in Anspruch 1 angegebenen Bedeutungen besitzen und X ein Halogenatom oder eine Sulfonatgruppe bedeutet, umsetzt.

**2.** Verfahren nach Anspruch 1, **dadurch gekennzeichnet,** daß man die Verbindungen herstellt, die der Formel I entsprechen, worin Z eine gegebenenfalls substituierte Phenylgruppe bedeutet.

**3.** Verfahren nach Anspruch 2, **dadurch gekennzeichnet,** daß man die Verbindungen herstellt, worin Z eine nichtsubstituierte Phenylgruppe bedeutet.

**4.** Verfahren nach Anspruch 1, **dadurch gekennzeichnet,** daß man die Verbindungen der Formel I herstellt, worin Z eine Pyridyl-, Pyrrolyl-, Furyl-, Thienyl- oder Imidazolylgruppe bedeutet.

**5.** Verfahren nach Anspruch 1, **dadurch gekennzeichnet,** daß man die Verbindungen der Formel I herstellt, worin Z eine Gruppe $OR_7$ bedeutet.

**6.** Verfahren nach Anspruch 5, **dadurch gekennzeichnet,** daß man die Verbindungen der Formel I herstellt, worin $R_7$ eine Alkylgruppe bedeutet.

**7.** Verfahren nach Anspruch 5, **dadurch gekennzeichnet,** daß man die Verbindungen der Formel I herstellt, worin $R_7$ eine $C_1$-$C_3$-Alkylgruppe bedeutet.

**8.** Verfahren nach einem der Ansprüche 1 bis 7, **dadurch gekennzeichnet,** daß man die Verbindungen der Formel I herstellt, worin A, B, C und D jeweils CH bedeuten.

**9.** Verfahren nach einem der Ansprüche 1 bis 7 zur Herstellung von Verbindungen der Formel I, worin eines der Symbole A, B, C oder D N bedeutet.

**10.** Verfahren nach Anspruch 9, **dadurch gekennzeichnet,** daß man die Verbindungen der Formel I herstellt, worin A N bedeutet.

**11.** Verfahren nach Anspruch 1, **dadurch gekennzeichnet,** daß man die Verbindungen der Formel I herstellt, worin $R_1$ und $R_2$ jeweils eine $C_1$-$C_6$-Alkylgruppe, $R_3$ eine $C_1$-$C_3$-Alkylgruppe, $R_{10}$ ein Wasserstoffatom und Z eine Gruppe $OR_7$ bedeuten, worin $R_7$ eine $C_1$-$C_6$-Alkylgruppe darstellt.

**12.** Verfahren nach Anspruch 11, **dadurch gekennzeichnet,** daß man die Verbindungen herstellt, worin $R_4$ Wasserstoff bedeutet.

**13.** Verfahren nach Anspruch 11, worin $R_4$ Wasserstoff bedeutet und das Symbol C substituiert ist.

**14.** Verfahren nach Anspruch 1, **dadurch gekennzeichnet,** daß man die Verbindungen der Formel I herstellt, worin $R_1$ eine Alkylgruppe und $R_2$ und Z jeweils eine gegebenenfalls substituierte Phenylgruppe bedeuten.

**15.** Verfahren nach Anspruch 1, **dadurch gekennzeichnet,** daß man die Verbindungen der Formel I herstellt, worin $R_1$ eine Alkylgruppe, $R_2$ und Z eine gegebenenfalls substituierte Phenylgruppe und $R_3$ und $R_{10}$ jeweils ein Wasserstoffatom bedeuten.

**16.** Verfahren nach einem der Ansprüche 14 und 15, **dadurch gekennzeichnet,** daß man die Verbindungen herstellt, worin die Symbole A, B, C und D jeweils CH bedeuten.

**17.** Verfahren nach einem der Ansprüche 14 und 15, worin das Symbol A N und die Symbole B, C und D CH bedeuten.

**18.** Verfahren nach Anspruch 1, **dadurch gekennzeichnet,** daß man [7-Chlor-4-(1-N,N-dipropylcarbamoylethylamino)-chinolin-3-yl]-carbonsäureethylester und dessen Additionssalze mit pharmakologisch annehmbaren Säuren herstellt.

**19.** Verfahren nach Anspruch 1, **dadurch gekennzeichnet,** daß man N-Methyl-N-(4-chlor)-phenyl-[3-benzoyl-7-chlor-chinolin-4-yl]-aminoacetamid und dessen Additionssalze mit pharmakologisch annehmbaren Säuren herstellt.

**20.** Verfahren nach Anspruch 1, **dadurch gekennzeichnet,** daß man N-Methyl-N-(4-chlor)-phenyl-(3-benzoyl-1,5 naphthyridin)-4-yl-aminoacetamid und dessen Additionssalze mit pharmakologisch annehmbaren Säuren herstellt.